## Europäisches Patentamt
## European Patent Office
## Office européen des brevets

(19)

(11) Veröffentlichungsnummer: **0 279 460**
**A1**

(12) **EUROPÄISCHE PATENTANMELDUNG**

(21) Anmeldenummer: **88102469.9**

(22) Anmeldetag: **19.02.88**

(51) Int. Cl.⁴: **C12N 15/00** , A61K 39/29 ,
C12Q 1/68 , G01N 33/576

(30) Priorität: **20.02.87 DE 3705512**
**24.12.87 DE 3744242**

(43) Veröffentlichungstag der Anmeldung:
**24.08.88 Patentblatt 88/34**

(84) Benannte Vertragsstaaten:
**AT BE CH DE ES FR GB GR IT LI LU NL SE**

(71) Anmelder: **Seelig, Renate, Dr.**
**Kriegstrasse 99**
**D-7500 Karlsruhe(DE)**

Anmelder: **Seelig, Hans Peter, Prof. Dr.**
**Kriegstrasse 99**
**D-7500 Karlsruhe(DE)**

Anmelder: **Burckhardt, Jean, Dr.**
**Kriegsstrasse 99**
**D-7500 Karlsruhe(DE)**

(72) Erfinder: **Seelig, Renate, Dr.**
**Kriegstrasse 99**
**D-7500 Karlsruhe(DE)**
Erfinder: **Seelig, Hans Peter, Prof. Dr.**
**Kriegstrasse 99**
**D-7500 Karlsruhe(DE)**
Erfinder: **Burckhardt, Jean, Dr.**
**Kriegsstrasse 99**
**D-7500 Karlsruhe(DE)**

(74) Vertreter: **Deufel, Paul, Dr. et al**
**Patentanwälte Müller-Boré, Deufel, Schön,**
**Hertel, Lewald, Otto Isartorplatz 6 Postfach**
**26 02 47**
**D-8000 München 26(DE)**

(54) **Virusantigen, Verfahren zu seiner Gewinnung und Anwendung in Diagnose und Therapie (Impfstoff).**

(57) Die Erfindung betrifft eine Non-A,Non-B-Hepatitis assoziierte DNA von etwa 5 KB, ein Verfahren zur Herstellung derselben nach an sich bekannten Methoden und die Verwendung dieser DNA oder von Fragmenten davon zur Diagnose von Non-A,Non-B-Hepatitis sowie zur Synthese von Proteinen zur Erzeugung von immunologischen Reagentien für den Nachweis von Non-A,Non-B-Hepatitis oder zur Erzeugung von Vaccinen. Die DNA und Fragmente derselben können geklont und auch in geeignete Vektoren eingesetzt werden, um Virusexpressionsprodukte zu liefern.

## Virusantigen, Verfahren zu seiner Gewinnung und Anwendung in Diagnose und Therapie (Impfstoff)

Non-A, Non-B-Hepatitiserkrankungen sind in der Literatur ausführlich und zahlreich beschrieben. Ebenso bekannt sind aber die Schwierigkeiten der Erfassung des NANBH-Virus und Nachweis der Krankheit.

Es wurden nun aus dem Stuhl von Non-A, Non-B-Hepatitispatienten Partikel isoliert und bezüglich ihres Molekulargewichts und ihrer Beschaffenheit charakterisiert sowie ein Nachweisverfahren auf das Vorhanden sein solcher Partikel gefunden und der Nachweis dieser Partikel zur Diagnose des Vorliegens einer Non-A, Non-B-Hepatitis bei leberkranken Patienten angewandt.

Aus den aus dem Stuhl isolierten Partikeln wurde DNA isoliert und nach herkömmlichen Methoden kloniert. Die so erhaltenen klonierten DNA-Stränge wurden zum Nachweis des Vorliegens homologer oder sehr ähnlicher DNA wiederum im Stuhl, Serum, Lebergewebe und Körperflüssigkeiten von leberkranken Patienten verwendet, bei denen auf diesem Wege eine Non-A, Non-B-Hepatitis mit hinreichender Wahrscheinlichkeit diagnostiziert werden kann.

Die Erfindung betrifft also zusammengefaßt die Isolierung Non-A, Non-B-Hepatitis-assoziierte Partikel, deren DNA sowie das Klonieren von DNA und die Verwendung sowohl dieser Partikel als auch der erhaltenen DNA-Klone zur Eingrenzung von Lebererkrankungen auf das Vorliegen einer Non-A, Non-B-Hepatitis. Weitere Aspekte der Erfindung liegen in der Entwicklung von erweiterten Nachweisverfahren und schließlich auch Impfstoffen auf Basis der zu erhaltenden Antikörper und durch Synthese synthetischer Peptide mit der Sequenz der Partikel, die diesen Virusproteinen entspricht und die sich aus der Sequenz der klonierten DNA vorhersagen läßt.

Aus den Stuhlproben Non-A, Non-B-Hepatitis erkrankter Patienten wurde eine Substanz isoliert, die sich signifikant ge häuft im Stuhl solcher Non-A-Non-B-Hepatitis-Patienten findet, dagegen bei gesunden, sowie bei Patienten mit Lebererkrankungen anderer Genese in signifikant geringerem Ausmaß findet. Ein Verfahren zum Nachweis dieser Substanz wurde entwickelt, worin Polystyrolbeads mit verdünntem Serum von Non-A, Non-B-Hepatitis-Rekonvaleszenten beschichtet werden. Die gewaschenen beads werden dann mit einer 10 %-igen Stuhlsuspension eines zu untersuchenden Probanden inkubiert, wobei evtl. vorhandene Non-A -Non-B-Hepatitis assoziierte Substanz an die in dem Rekonvaleszentenserum enthaltenen, an die Polystyrolkugeln gebundenen Antikörper gegen diese Substanz bindet und so immobilisiert wird. Die Bindung der Non-A, Non-B-Hepatitis assoziierten Teilchen kann nachgewiesen werden durch Bindung von menschlichem IgG wiederum aus Rekonvaleszentenserum von Non-A-Non-B-Hepatitis-Patienten, das mit Jod 125 radioaktiv markiert ist. Bei Vorliegen der Non-A, Non-B-Hepatitis-assoziierten Substanz im Stuhl des Probanden wird radioaktives Immunglobulin an diese gebunden und das entsprechende Signal zum Nachweis verwendet.

Bei Verwendung dieses Nachweisverfahrens für die Hepatitis-Non-A-Non-B-assoziierte Substanz in großen Kollektiven von Patienten mit Lebererkrankungen unterschiedlicher Genese zeigte es sich, daß diese Substanz hochsignifikant gehäuft bei Patienten mit gesicherter Hepatitis Non-A, Non-B im Stuhl nachweisbar ist (s. Tabelle 1). Bei Patienten mit anderen Lebererkrankungen, bei denen eine Non-A, Non-B-Hepatitis eher unwahrscheinlich ist, die teilweise in ihrem klinischen Bild jedoch einer akuten oder abgelaufenen Non-A, Non-B-Hepatitis ähneln können, insbesondere auch bei Patienten mit einer akuten unabgelaufenen Hepatitis A oder B fand sich dagegen nur in äußerst wenigen Fällen die Non-A, Non-B-Hepatitis-assoziierte Substanz (s. Tabellen 2 und 3). Hieraus ergibt sich, daß das Vorliegen dieser Substanz einen deutlichen Hinweis auf die Ätiologie einer zunächst unbe kannten, entzündlichen Lebererkrankung liefern kann, und daß der Nachweis dieser Substanz daher eine Untersuchung von hohen diagnostischem Wert zum Nachweis oder Ausschluß des Vorliegens einer Non-A, Non-B-Hepatitis liefern kann.

Die mit der Non-A, Non-B assoziierte Substanz weist somit eine hohe Affinität gegenüber menschlichen Immunglobulinen und Fibronectin sowie dessen nicht kollagenbindenden Spaltprodukten auf. Die Bindung an Fab-2-Bruchstücke aus dem IgG gesunder und Hepatitis Non-A, Non-B-rekonvaleszenter Personen spricht gegen eine unspezifische Bindung der assoziierten Substanz an dem Fc-Teil der Immunglobuline. Die hohe Affinität gegenüber Fibronectin und dessen nicht kollagenbindenden Spaltprodukten ist eine Eigenschaft, die diese Substanz mit antigenen Proteinen anderer Viren gemeinsam hat (Seelig und Mitarbeiter 1983). Behandlung mit organischen Lösungsmitteln (Chloroform, Äther) und mit Hitze (70°C, 10 min.) zerstört die Bindungsaffinität der Non-A, Non-B assoziierten Substanz nicht. Während die Verdauung mit Chymotrypsin, Trypsin, Elastase und Neuraminidase keinen Einfluß auf die Bindungseigenschaften der Substanz zeigt, führt die Verdauung mit Papain zu einem vollständigen und schnellen Verlust der Bindungsaffinität.

Nach Zentrifugation bei 150.000 g, 2 Std., läßt sie sich im Sediment nachweisen und stellt sich nach 72-stündiger Laufzeit bei einer Dichte von 1,3 (1,29 - 1,32) g/ml Cäsiumchlorid ein. Nach Auftrennung der bei 1,30 g/ml Cäsiumchloridbandenden Fraktion über eine Gradienten-Polyacrylamidgel-Elektrophorese werden in der Silberfärbung mehrere Bande unterschiedlichen Molekulargewichts dargestellt. Nach Transfer auf Nitrocellulose lassen sich im Western-Blot mit radioaktiv markierten IgG und Fab-2-Fragmenten von Non-A, Non-B Hepatitis-Patienten und gesunden Probanden Bande darstellen, die bei Extraktion gesunder Kontrollstühle nicht auftreten. Insgesamt werden 4 Banden dargestellt, zwei gut sichtbare Hauptbande mit einem geschätzten Molekulargewicht von ca. 64.000 und 56.000 sowie zwei schwächere Bande mit einem geschätzten Molekulargewicht von 51.000 und 43.000. Die Banden mit Fab-2-Bruchstücken sind schwächer und zeigen einen höheren Background. Die Auftrennung von Rohstuhlkonzentraten ohne Cäsiumchlorid-Reinigung nach SDS-Page und Blotting zeigten in positiven Stühlen die beiden Hauptbande mit einem ungefähren Molekulargewicht um 60.000.

Bei weiterer Analyse der Non-A, Non-B-Hepatitis-assoziierten Partikel ließ sich DNA isolieren und mit herkömmlichen Methoden in Fragmenten klonieren. Diese klonierten DNA-Fragmente aus dem Stuhl von Non-A, Non-B-Hepatitis-Patienten konnten als DNA-Sonden für die Untersuchung des Stuhls, Serums, Lebergewebe und Körperflüssigkeiten sowie Blutkonserven und Plasmaderivaten anderer Patienten mit Verdacht auf das Vorliegen einer Non-A, Non-B-Hepatitis verwendet werden. Mit den klassischen Hybridisierungsverfahren läßt sich so zeigen, ob in den unbekannten Stühlen DNA vorliegt, deren Sequenz der Non-A, Non-B-Hepatitis-assoziierten DNA der erhaltenen Klone so ähnlich ist, daß sich ein Hybridisierungssignal zeigt. Mit diesem weiteren Nachweisverfahren für Hepatitis Non-A, Non-B-assoziierten DNA-Sequenzen konnten dann Proben von Probanden untersucht werden auf das Vorliegen von Non-A, Non-B-Hepatitis-assoziierter DNA. Die bisherigen Ergebnisse legen nahe, daß es sich bei der Non-A, Non-B-Hepatitis-assoziierten Substanz um ein Viruspartikel handelt und daß es sich bei der isolierten DNA-Sequenz um eine Sequenz einer Virus-DNA handelt, wie das Aufschlußverfahren einerseits und andererseits die Sequenz selbst zeigen.

Die folgenden Beispiele erläutern die Erfindung.

Beispiel 1

Isolierung der Non-A-Non-B-Hepatitis assoziierten Substanz aus Patientenstuhl.

Verwendeter Puffer: Tris-HCl, pH 7,4; 0,05 N in allen Arbeitsschritten

a) Aufarbeitung:

0,5 g Stuhl werden in 10 ml Puffer suspendiert, bei 8.000 g zentrifugiert und der Überstand gesammelt. Der Rückstand wird noch einmal mit 10 ml und anschließend mit 5 ml Puffer ausgewaschen. Die gesammelten Überstände werden zentrifugiert (30 min., 10.000 rpm) und durch ein bakteriendichtes Filter filtriert (Millipore 0,22 um). Der Überstand wird mit PEG 6000 (Endkonzentration 10% bzw. 0,4 mol/l) präzipitiert. Nach mindestens 2 und höchstens 12 Stunden wird das Präzipitat abzentrifugiert und in 10 ml Puffer gelöst. Diese Lösung wird mit 10 ml Freon ausgeschüttelt, die Phasen durch Zentrifugation getrennt, die Freonphase noch einmal mit 5 ml Puffer gewaschen. Die Fällung mit PEG und NaCl (Endkonzentration 10% bzw. 0,4 mol/l) wird wiederholt, das Präzipitat in ca. 800 ul Puffer aufgenommen.

Die so erhaltene Lösung wird mit RNase und DNase 1 Std. bei 37°C verdaut (800 ul PEG-Präzipitat in Tris-HCl-Puffer, pH 7,4; 0,05 M; 2.800 U RNase und 1.500 U DNase, proteasenfreie Präparationen, Boehringer). Isolierungen, die nicht zur Extraktion von DNA dienen, können ohne diesen Schritt durchgeführt werden.

Nach Verdauung mit DNase und RNase wird das Inkubat auf einen Cäsiumchlorid-Gradienten gebracht.

b) Isolierung der Non-A, Non-B assoziierten Substanz über einen Cäsiumchlorid-Gradienten.

Die Zentrifugenröhrchen werden mit 1 ml Cäsiumchlorid-Lösung mit einer Dichte von 1,4 g/ml gegeben und mit je 3 ml Cäsiumchlorid von einer Dichte von 1,3 g 1,25 g und 1,2 g/ml überschichtet. Alle Cäsiumchlorid-Lösungen werden mit dem oben genannten Puffer hergestellt, der Gradient wird mit 800 ul Stuhlextrakt überschichtet, die Zentrifugationsdauer beträgt 65 - 72 Stunden. Temperatur + 10°C, rpm 31.000. Nach Beendigung der Laufzeit wird der Gradient im unteren Bereich (Dichte 1,2 - 1,4) in Fraktionen von ca. 200 ul gesammelt, im oberen Bereich Dichte 1,1 - 1,2 können größere Fraktionen entnommen werden. Die Dichte jeder Fraktion wird durch Messung des Brechungsindex bestimmt. Alle Fraktionen werden ausgiebig gegen Puffer dialysiert und 50 µl jeder Fraktion in NANB-Assay auf Non-A, Non-B assoziierende Substanz untersucht. Von den positiven Fraktionen wird die Eiweißkonzentration nach Lowry bestimmt.

## Verifizierung und Charakterisierung der Substanz

### c) Herstellung einer Gradienten-Page

Zwei Lösungen mit verschiedenen Acrylamid-konzentrationen werden durch einen Gradientenmi-scher zu einem linearen Gradienten aufgeschichtet. Die Lösung mit der höheren AA-Konzentration enthält außerdem 15 % Saccharose, um Turbulenzen zu verhindern. Ansonsten sind die Lösungen zusammengesetzt wie bei LAEMMLI (1970) beschrieben. Dies gilt auch für das Kammgel.

Die dialysierten und evtl. eingeengten Fraktionen des Cäsiumchlorid-Gradienten werden mit 10 $\mu$l SDS, 10 $\mu$l Glycerin und 5 $\mu$l beta-Mer-captoäthanol, 5 $\mu$l Bromophenolblau pro 100 $\mu$l Probe versetzt und 3 Min. im Wasserbad gekocht. Danach werden sie mit 5 $\mu$l 0,1%igem Pyronin versetzt und auf das Gel aufgetragen. Laufzeit 3 1/2 Stunden, Spannung 160 - 300 V, Stromstärke 40 - 25 A, Leistung 20 W.

Ca. 5 Min. vor Ende des Laufes wird noch einmal 5 $\mu$l Pyronin aufgetragen und der Lauf beendet, sobald Pyronin das Kammgel durchlaufen hat. Das Gel wird entweder mit Silber gefärbt (WRAY und Mitarbeiter 1981) oder ein Western-Blotting durchgeführt (TOWBIN und Mitarbeiter 1979). Das Blotting wird über Nacht bei 0,5 A, anschließend 1 Std. bei 1 A durchgeführt.

### d) Behandlung von Western-Blots

Nach Beendigung des Blotting werden die verschiedenen Streifen (an den Pyroninmarkierungen kenntlich) ausgeschnitten und mitgeführte Molekulargewichtstandards mit Amido schwarz gefärbt. Probenstreifen werden 24 - 72 Stunden in 1%iger Gelatine PBS-Lösung geschüttelt. Die IgG-Fraktion eines Patienten bzw. die Fab-2-Fragmente dieser IgG-Fraktion werden mit Jod 125 (Chloramin T) markiert: 0,5 mCi auf 100 ug Protein. Dabei werden etwa 70 % der Aktivität inkorporiert. Der Tracer, dessen Volumen ca. 2 ug Protein entsprechen sollte, wird in 20 ml Gelatine/PBS verdünnt und damit die Streifen 12 Stunden unter Schütteln inkubiert. Danach werden die Streifen je 1 Stunde 3 $\times$ mit Gelatine-PBS, 2 $\times$ mit PBS-Tween 0,5 % und 1 $\times$ mit Wasser gewaschen. Nach Trocknen der Streifen werden diese auf einen empfindlichen Röntgenfilm aufgelegt und bei -70°C 2 - 7 Tage exponiert.

## Beispiel 2

Nachweismethode für HNANB-assoziierte Substanz im Stuhl

Polystyrol Beads (Plasticball company, Chicago) wurden mit Serum von rekonvaleszenten Patienten mit Non-A, Non-B-Hepatitis in einer Verdünnung von 1:200 in Carbonatpuffer, pH 9,2, 0,01 Mol/l, 12 Stunden bei Raumtemperatur inkubiert. Die so beschichteten beads wurden mit phosphatgepufferter physiologischer Kochsalzlösung (PBS) ausgiebig gewaschen. Die Stuhlproben wurden in Form einer 10 %-igen Stuhlsuspension (g/V) 2 Stunden bei 37°C mit den wie oben geschichteten Polystyrol Beads inkubiert, danach ausgiebig mit PBS, die 0,5 % Tween 20 enthielt, gewaschen und danach 1 Stunde mit humanem IgG aus dem Serum von Rekonvaleszenten von einer Non-A, Non-B-Hepatitis, das mit Jod 125 markiert war, bei 37°C inkubiert. Nach neuerlichem, ausführlichem Waschen mit destilliertem Wasser wurden die an die Beads gebundene Radioaktivität in einem Gamma-Counter ausgezählt. Stuhlproben, bei denen die gebundene Radioaktivität den dreifachen Wert der Negativkontrolle erreichte, wurden als positiv für das Vorliegen der Non-A, Non-B-Hepatitis-assoziierten Substanz befunden.

Jede der beigefügten Tabellen 1 bis 4 wurde wie oben beschrieben erstellt und nach den angegebenen Kriterien ausgewertet. Es wurde festgestellt, daß gemäß Tabelle 1 bei Patienten einer gesicherten Non-A, Non-B-Hepatitis diese Substanz in einem großen Prozentsatz der Fälle gefunden wurde,
daß gemäß Tabelle 2 bei Verwendung dieses Essays für diese Non-A, Non-B-Hepatitis-assoziierte Substanz bei einem Patientenkollektiv mit einer Vielzahl von ganz unter schiedlichen Lebererkrankungen, die aber nichts mit einer Non-A, Non-B-Hepatitis zu tun haben, die Substanz , wenn überhaupt, nur in sehr niedrigen Prozentsätzen gefunden wird.

Tabelle 3 zeigt, daß man während der Untersuchung von Patientenkollektiven mit Hepatitis anderer Genese, also entweder Hepatitis A oder Hepatitis B zu einem sehr niedrigen Prozentsatz die Non-A, Non-B-assoziierte Substanz findet.

Es ist festzustellen, daß man bei den Non-A, Non-B-Hepatitis-Fällen praktisch immer, also fast 30% positive Befunde hat, wohingegen bei den Hepatitis A, Hepatitis A-Verdacht, Hepatitis B und Posthepatitis B Patienten die Zahlen erheblich niedriger liegen.

Die relative hohe positive Anzahl für die Substanz bei chronischen Hepatitis B-Patienten läßt vermuten, daß es sich um eine Doppelinfektion mit Non-A, Non-B und Hepatitis B handelt.

Tabelle 4 zeigt folgendes: Eine Untersuchung an Empfängern mit einer Bluttransfusion, die prinzipiell als Risikopatienten zu gelten haben, weil bei Blutransfusionen häufig eine Non-A, Non-B-Hepatitis Infektion eintritt. Es handelt sich um eine prospektive Studie.

Hieraus ergibt sich sehr deutlich, daß abhängig vom Schweregrad der Folgen der Transfusion einerseits Patienten gar nichts geschehen ist, wo also diese Substanzen nur in sehr geringem Maße ausgeschieden worden ist, andererseits bei Patienten, bei denen die Krankheit erkennbar ist und bei denen, die eine ganz manifeste Hepatitis haben, in über 70 % der Fälle diese Substanz nachweisbar ist.

Beispiel 3: Isolierung von DNA aus dem Stuhl und Klonierung von DNA-Sequenzen und deren Einbau in entsprechende Vektoren.

Einbau dieser DNA in Klonierungsvektoren.

Aus dem Stuhl von Patienten mit Non-A, Non-B-Hepatitis wurden, wie in Beispiel 1 beschrieben, Non-A, Non-B-Hepatitis-assoziierte Partikel isoliert und, wie dort beschrieben, mit RNAs und DNAs behandelt und über einen Cäsiumchlorid-Gradienten gereinigt und ausgiebig dialysiert. Die dialysierten Fraktionen wurden auf die oben beschriebene Art auf die Anwesenheit von NANB-assoziierter Substanz untersucht. Die Fraktion der Dichte von 1,3 g/ml im Cäsiumchloridgradienten wurde mit 50 Mikrogramm Proteinase K, 1 % SDS und EDTA in einer Endkonzentration von 10 mM/l 6 Stunden bei 37°C verdaut. Proteine wurden durch Extraktion mit 80 %-igem Phenol (Gewicht/Volumen) und Chloroform entfernt, und daraufhin die in der wässrigen Phase gelöste DNA in Gegenwart von 0,3 Mol/l Natriumacetat mit einem zweieinhalb-fachen Volumen an Äthanol für 60 Stunden bei -70°C gefällt. Danach wurde zentrifugiert und das Sediment einmal mit 70 %-igem Äthanol gewaschen, getrocknet und daraufhin in TE-Puffer bestehend aus 10 mmol/l Tris, pH 8,0, und 1 mmol/l EDTA, in einem Volumenverhältnis von einem Mikroliter/5 mg aufgearbeitetem Stuhl, aufgenommen.

15 Mikroliter dieser DNA-Stammlösung wurden in einem Gesamtreaktionsvolumen von 50 Mikroliter mit 6 Einheiten Klenow-Polymerase (DNA-Polymerase I, großes Fragment), 1 Mikroliter einer Lösung von dATP , dTTP und dGTP, jeweils in einer Konzentration von 1 mM/l, sowie 5 Mikroliter alpha-32P-dCTP (3.000 Ci/mmol, 10 mCi/ml) im Inkubationspuffer für Klenow-Polymerase nach den Angaben des Herstellers (Boehringer, Mannheim) 1 Stunde bei Raumtemperatur inkubiert.

Danach wurden 2,5 Mikroliter einer 1 mMol/l dCTP-Lösung zugegeben und die Probe eine weitere Stunde bei Zimmertemperatur inkubiert. Nach Inaktivierung des Enzyms durch Erhitzen auf 68°C für 10 Minuten wurde die Reaktionslöung auf 0°C abgekühlt. Danach wurde das Reaktionsgemisch zusammen mit 2 Einheiten T4-DNA-Ligase, 1 Mikrogramm phosphorylierter EcoR-I-Linker und 6 Mikroliter 10 mM ATP-Lösung für 16 Stunden bei 16°C inkubiert. Das Reaktionsgemisch wurde danach auf eine Endkonzentration von 150 mMol/l NaCl eingestellt und mit 240 Einheiten EcoR-I Restriktionsendonuclease (80 Einheiten/Mikroliter) 3 Stunden bei 37°C verdaut. Die Reaktion würde durch Zugabe von 5 Mikroliter 80 %-igem Phenol und 1 Mikroliter 20 %-igem SDS gestoppt. Die radioaktiv markierte und mit Linkern versehene DNA wurde von Salz und nicht legierten Linkern über eine Sepharose 4B-CL-Säule (3 ml Säulenvolumen, 25 cm Länge) abgetrennt und mit 2,5 Volumina Äthanol 16 Stunden bei -70°C gefällt. Die präzipitierte DNA wurde 10 Min. bei 14.000 g zentrifugiert und das Sediment bei 150 Mikroliter 70 %-igem Alkohol gewaschen, getrocknet und in 10 Mikroliter TE-Puffer aufgenommen.

Beispiel 4: Einbau der isolierten DNA in lambda-Phagen und Transfektion auf Bakterien.

Zur Ligation mit der Vektor-DNA wurden 2 Mikrogramm DNA des Phagen-lambda 1149 mit 2 Einheiten EcoR-I (4 E/Mikroliter) in einem Gesamtreaktionsvolumen von 6 Mikroliter (Inkubationspuffer nach Angabe des Enzymherstellers (Boehringer, Mannheim) 1 Stunde bei 37°C inkubiert. Dann wurde das Enzym durch 10-minütiges Erhitzen auf 68°C inaktiviert. Diese Lösung wurde mit 3 Mikroliter markierter DNA, die wie im vorigen Beispiel beschrieben, isoliert wurde, 1 Mikroliter 5 mM ATP und 1 unit T4-DNA-Ligase bei Raumtemperatur ligiert. 4 Mikroliter dieses Reaktionsansatzes wurden in lambda-Phagenhüllen verpackt (unter Verwendung eines fertigen Verpakkungsextrakts der Firma Giga-Pack, Vector-cloning systems). Mit diesen verpackten Phagen wurde der Escherichia Coli Stamm NM 514 infiziert. Zum screening auf das Vorhandensein eingebauter DNA wurden ca. $10^5$ "plaque forming units" auf 22 × 22 cm screening plates der Firma Nunc ausplattiert und über Nacht bei 37°C inkubiert. Phagen-DNA wurde auf Nitrozellulosefilter durch Abklatsch übertragen und die so erhaltenen Abklatschfilter wurden mit 1 Mikroliter der oben beschriebenen radioaktiven DNA Stammlösung gemäß Maniatis et al. (1982) hybridisiert, gewaschen und 6 Std. bei 70°C autoradiographiert.

Von 70 positiven Hybridisierungssignalen wurden 17 zugeordnete Phagenkolonien in einer Dichte von ca. 5 Plaques pro cm² ausplaziert. Nach plaques purification nach BENTON und DAVIS (1978) wurden von den schließlich erhaltenen 16 positiven Plaques Lysate hergestellt. Mehrere dieser Phagen enthielten DNA-Inserte in einer Länge zwischen 0,3 und etwa 1,5 KB, die mit einer Probe der ursprünglichen DNA-Stammlösung hybridisierten.

Beispiel 5: Charakterisierung eines DNA-Fragmentes von 0,45 KB und Subklonierung dieses Fragmentes in einem Plasmid PUC 19 in Escherichia coli.

Das DNA-Fragment wurde durch Verdauung mit ·entsprechenden Restriktionsendonucleasen (EcoR-I) unter den oben bereits beschriebenen Bedingungen aus dem der Phagen DNA entfernt, das Fragment dann auf Agarose Gel von der lambda-Phagen DNA getrennt und die dem Insert entsprechende DNA-Bande aus dem Gel ausgeschnitten und eluiert. Die solchermaßen isolierte Insert-DNA wird dann genau wie oben beschrieben, in Vektor-DNA PUC 19 hineinligiert und dann auf Escherichia coli Stamm DH 1 transfiziert. Nach Selektion von Bakterienstämmen, die dieses Plasmid mit dem eingebauten Hepatitis Non-A, Non-B-DNA-Insert aufgenommen haben und vermehren wurde die Züchtung dann durchgeführt und wie bei T. Maniatis et al. 1982, in "Molecular Cloning, a laboratory manual," beschrieben, aufgearbeitet.

Das gleiche Insert wurde radioaktiv markiert und mit Southern-Analyse auf Hybridisierung mit dem DNS-Ausgangsmaterial, DNS-Extraktionen aus Stühlen von Kontrollpersonen, Hepatitis B-Virus-DNS und Escherichia coli Plasmid PBR 322 geprüft.

Beispiel 6: Nachweis von Non-A, Non-B-assoziierter DNA im Serum

2 - 5 ml Serum werden bei 150.000 g 2 Stunden zentrifugiert. Das Sediment wird in 200 ul Proteinase K-Lösung (0,5 mg/ml + 10 mmol EDTA) bei 37°C 1 Stunde verdaut. Danach werden zu dieser Lösung 165 $\mu$l einer heiß gesättigten Natriumjod Lösung (2,5 g Natriumjodid/ml H₂O, 75°C) gegeben (Endkonzentration 12,5 M). Diese Lösung wird 10 Minuten auf 90°C erhitzt und unmittelbar durch Nitrozellulosefilter mittels Vakuum filtriert (Dot-Blot-Apparatur, Schleicher + Schüll). Nach der Filtration wird die Nitrozellulosefolie 3 × mit 70%igem Äthanol gewaschen und darauf 10 Min. bei Raumtemperatur in 100 ml Essigsäureanhydrid (100 ml 0,1 M Triäthanolamin + 250 ul Essigsäureanhydrid) inkubiert. Nach der Inkubation wird die Folie im Vakuum bei 80°C 1 - 2 Stunden gebacken. Die trockene Folie wird in Vorhybridisierungslösung gebracht und 3 Stunden bei 65°C inkubiert (6 × SSC, 1 × Denhardt, 0,5 % SDS, 20 $\mu$g/ml Hitze denaturierte Heringssperma-DNS entsprechend den Angaben in: Maniatis, Molecular Cloning, 1982). Mittels Nick-Translation (siehe Maniatis, Molecular Cloning, 1982) mit 32P-markierte Probe über Nacht unter Prähybridisierungsbedingungen inkubiert. Danach wird die Nitrozellulosefolie 3 × gewaschen (1) 6 × SSC, 1 × Denhardt, 0,5 % SDS, 20 $\mu$g/ml Heringssperma-DNA, 2). 1 × SSC, 0,5 % SDS, 1 × Denhardt, 3). 0,1 × SSC + 0,05 % SDS). Nach Trocknen der Folie wird diese auf einen Röntgenfilm (Kodak X-Omat) aufgelegt (Autoradiographiezeit 6 Stunden bis 2 Tage bei -70°C).

Bei Seren mit hohem Gehalt an HNANB-Viren kann die Ankonzentrierung von 2-5 ml Serum durch Zentrifugation entfallen und die Seren nach Proteinase-K-Verdauung entsprechend der Methode (Seelig et al., klinikarzt 2/1985, Seite 86 ff) direkt auf Nitrozellulose aufgetragen werden. (s. Bsp. 7)

Beispiel 7: HNANB-DNA-Nachweis im Serum

200 ul Serum werden mit 75 ul Proteinase K-Lösung (Boehringer) (4 mg/ml) und 25 ul 0,5 M EDTA bei 37°C 1 Stunde inkubiert. Das Inkubat wird mit 100 ul 1 N NaOH 10 Minuten bei Raumtemperatur inkubiert und mit 250 ul 2 M NH₄OAc neutralisiert. 500 ul des Ansatzes (entsprechend 181 ul Serum) werden auf Nitrozellulose aufgetragen (Dott-Blott-Apparatur Schleicher + Schüll). Nach nochmaliger Neutralisierung mit 250 ul 2 M NaH₄OAc werden die Filter in Vakuum bei 80°C 45 Minuten getrocknet. Die Filter werden in 6 × SSC *, 0,5 % SDS **, 1 × Denhardt-Lösung *** und 20 ul/ml Hitze denaturierter (5 Minuten, 100°C)

* 20 × SSC (Standard Saline Citrat) entspricht 3 M NaCl, 1,5 M Natriumcitrat.

** SDS Natriumdodecylsulfat.

*** 100 × Denhardt-Lösung entspricht 2 % Ficoll; 2 % Polyvinylpyrolidon, 2 % Bovin Serumalbumin.

Heringsperma-DNA bei 65°C 3 Stunden prähybridisiert. Zur Hybridisierung die über Nacht unter Prähybridisierungsbedingungen erfolgt, wird das durch Nick-Translation mit 32P-markierten Inserts der Phagenclone verwendet (spezifische Aktivität ca. 1 - 2 $\times$ 10$^9$ cpm/ug DNA). Die Filter werden danach jeweils einmal 20 Minuten bei 65°C mit 6 $\times$ SSC, 1 $\times$ Denhardt-Lösung, 0,5 % SDS, 20 ug/ml denaturierter Heringssperma-DNA, danach mit 1 $\times$ SSC, 0,5 % SDS 1 $\times$ Denhardt-Lösung und 0,1 $\times$ SSC, 0,05 % SDS gewaschen und bei 80°C getrocknet. Die Autoradiographie erfolgt mittels Röntgenfilm und Verstärkerfolie (intensiv fying screens Kodak) mit einer Autoradiographiezeit von 6 - 48 Stunden bei -70°C.

### Beispiel 8

Dieses Beispiel zeigt eine einfachere Variante bezüglich der Probenvorbereitung zum Dot Blot:

1 ml Serum wird mit 350 $\mu$l Proteinase K-Lösung (3 mg/ml Proteinase K, 10 mM TRIS pH 7,5, 0,5 mM EDTA, 0,5 % SDS) und 125 $\mu$l 0,5 M EDTA 30 Min. bei 37°C inkubiert. Darauf wird die Lösung mit 8 ml 2 M TCA (pH 7,0), 3,2 ml 2 MNH$_4$A$_c$, 20 $\mu$l t RNA (10 mg/ml) und 5,3 ml H$_2$0 auf 16 ml verdünnt und die DNA mit 10 ml Isopropanol bei Raumtemperatur für 30 Min. gefällt. Die DNA wird abzentrifugiert (15 Min. bei 8000rpm), das Pellet mit 2 ml 70 % Ethanol gewaschen und in 600 $\mu$l 10 mM TRIS pH 8,4 und 1 mM EDTA aufgenommen, einmal mit Phenol, einmal mit Chloroform extrahiert und nochmals gefällt. Aliquots der gefüllten DNA können für die Dot Blots oder für Restriktionsanalysen eingesetzt werden.

Die Darstellung von partikelassoziierter DNA aus Stuhlproben erfolgt in gleicher Weise. Stuhlsuspensionen werden allerdings vorher steril filtriert und mit PEG gefällt.

### Beispiel 9

Wenn nur relativ wenig der N-A, N-B-assoziierten Substanz vorliegt, hat es sich als zweckmäßig erwiesen, die DNA durch Amplifizieren nachzuweisen:

Der Nachweis von Non-A, Non-B-DNA im Serum kann zweckmäßig durch Hybridisierung mit radioaktiv markierter klonierter Non-A, Non-B-DNA-Probe nach vorangehender spezifischer enzymatischer DNA-Amplifizierung nach bekannter Methode (SAIKI et al., Science 230, 1350, 1985) erfolgen.

25 $\mu$l Serum + 50 $\mu$l NaJ (gesättigte Lösung) mischen und 2 Min. bei 37°C inkubieren, danach 10 Minuten bei 0°C inkubieren. Das Inkubat wird auf eine Polycarbonatmembran (z.B. Uni Pore von Firma BioRadLab) gegen TE-Puffer (10 mmol Tris; 1mmol EDTA) 20 Min. dialysiert. 5 $\mu$l des Dialysats werden für die spezifische enzymatische DNA-Amplifikation entnommen. Die Amplifikation erfolgt nach bekannter Methode (SAIKI et al. 1985) mit Hilfe von je 0,5 $\mu$g Oligoprimer (Paar A und B bzw. C und D). Die Oligoprimer-Paare werden nach bekannten Sequenzierungsdaten mittels eines DNA-Synthesizers (Applied BioSystem 381) hergestellt. Nach Amplifizierung wird das Reaktionsgemisch auf einem 2%igen Agarosgel elektrophoretisch aufgetrennt, danach auf eine Nylonmembran (Genofit) transferiert und mit einer nach FEINBERG et al. 1983 (Annal. Biochem. 132, 6-13) mit 32P markierten klonierten HNANB-DNA-Probe hybridisiert. Nach Autoradiographie werden positive Resultate anhand mitgeführter Standards und Längenmarker identifiziert.

### Beispiel 10

Nachweis von DNA in geprüft infektiösem Plasmaderivat: Renger F. et al. veröffentlichten in "Deutsches Gesundheitswesen" Vol. 36, S. 560-563 (1981) eine Studie über eine kontrollierte Hepatitis Non-A, Non-B-Epidemie, ausgelöst durch die Applikation infektiöser Antirhesusfaktor D Immunglobulinpräparate. Die Herkunft dieser kontaminierten Präparate konnte vollkommen aufgeklärt werden. Nach Applikation dieses anti-D-Präparates erkrankten 79 % von 116 immunisierten - schwangeren Frauen. Dieses anti-D-Präparat (Charge I Ampulle A und C) sowie eine Charge mit niedriger Infektiosität (Charge II Ampulle B und D), die durch das selbe Säulensystem gereinigt wurde, standen zusammen mit Kontrollpräparationen (Gammavenin, Endobolin, Rhesonativ) zur Verfügung. Diese Präparationen wurden zusammen mit Pufferkontrollen, Hepatitis B-Virus-haltigem Serum, Positivkontrollen mit clonierten Virus-DNA-Fragmenten eingesetzt. Der Nachweis erfolgte mittels DNA-Amplifikation durch die Primer 237-238 (entstammen dem 0,4 Kb EcoRI-Fragment).

Ergebnisse:

In mehrfachen, unter verschiedenen Bedingungen durchgeführten Experimenten, fand sich nach Amplifikation ein starkes Signal in der Charge I (Ampulle A und C), ein schwaches Signal bei guter Amplifikationsausbeute in der Charge II (Ampulle B und D). Die drei Kontroll-Lyophilisate Gammavenin, Endobolin und Rhesonativ ergaben kein Signal. Der Zusatz von Hepatitis B-Virus-Genomspezifischen Primern zu den verschiedenen Gammaglobulin-Präparationen ergab ebenfalls keinen Hinweis auf die evtl. Kontaminierung mit Hepatitis B-Genom. Die mitgeführten Kontrollen dienten der Beurteilung der Effizienz der Amplifikation sowohl mit HBV-spezifischen Primern als auch mit Non-A, Non-B-spezifischen Primern. Der Nachweis der Identität der in der infektiösen Charge befindlichen DNA mit der DNA aus den Feces eines Patienten mit Hepatitis Non-A, Non-B sprechen für die Identifizierung einer in Hepatitis Non-A, Non-B-implizierten DNA.

Die Untersuchung von weiteren 57 Stuhlproben von Patienten mit sporadischer und posttransfusioneller HNANB mittels Radioimmunoassey wie in den vorhergehenden Beispielen beschrieben und im Dot-Blot-Verfahren, ergab eine signifikante Korrelation der beiden Untersuchungsverfahren hinsichtlich nachweisbarer HNANB-assoziierten Substanz (RIA) und nachweisbarer DNA (Dot-Blot, siehe Beispiel 7).

Die beigefügten Abbildungen erläutern die Erfindung:

Fig. 1 ist die Sequenz des Genoms

Fig. 2 ist eine Übersicht über die Schnittstellen mit EcoRI sowie die offenen Leserahmen und Leseraster

Fig. 3 und

Fig. 4 zeigen den Plus-und Minusstrang einer ca. 0,3 Kb Sequenz, also einer Teilsequenz des Genoms

Fig. 5 zeigt den offenen Leserahmen, und zwar die Leseraster 1A, 2A und 3A, und

Fig. 6 zeigt den offenen Leserahmen für die Leseraster 1B, 2B und 3B.

Die Sequenzierung der in den Figuren beigefügten Sequenzen erfolgte nach der Sanger-Methode nach Umklonieren von PUC 8 in M 13 bzw. Bluescript Vektor.

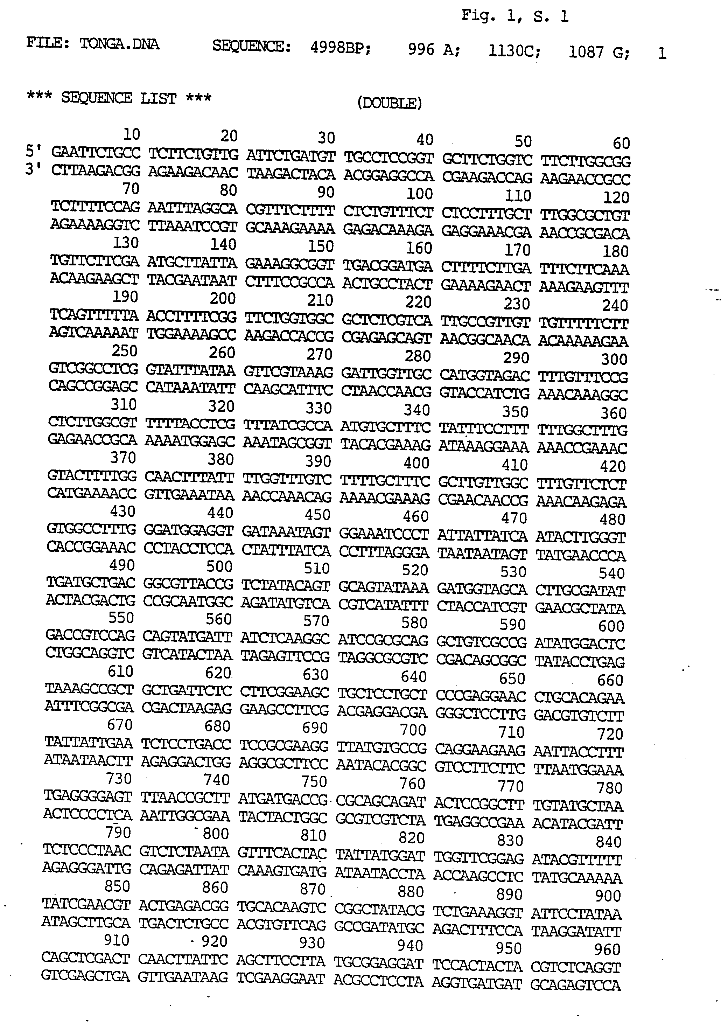

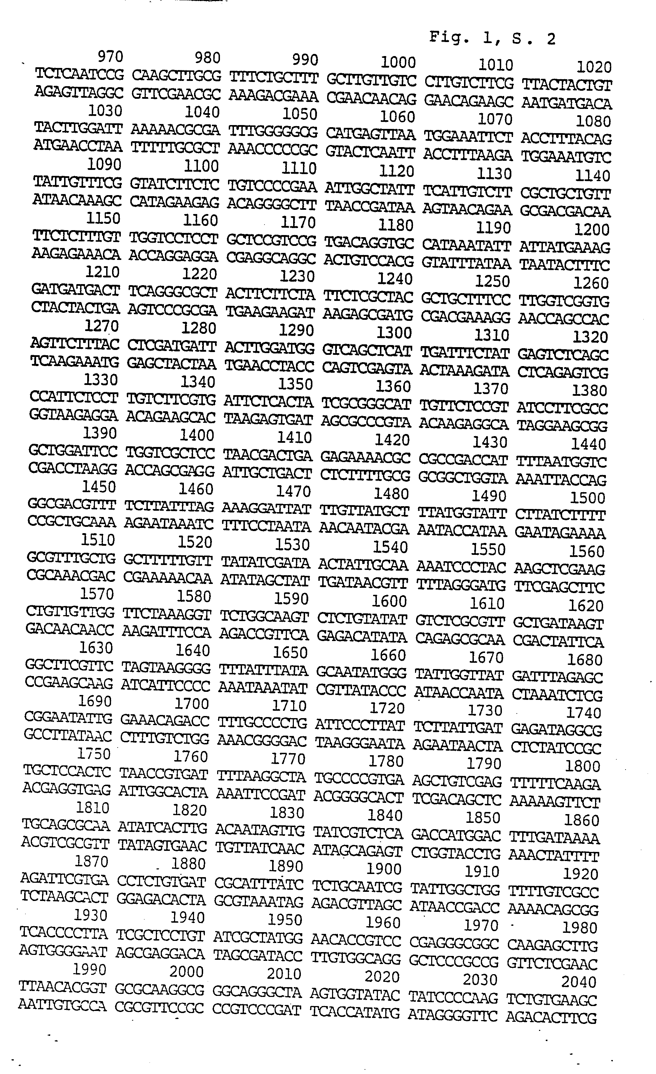

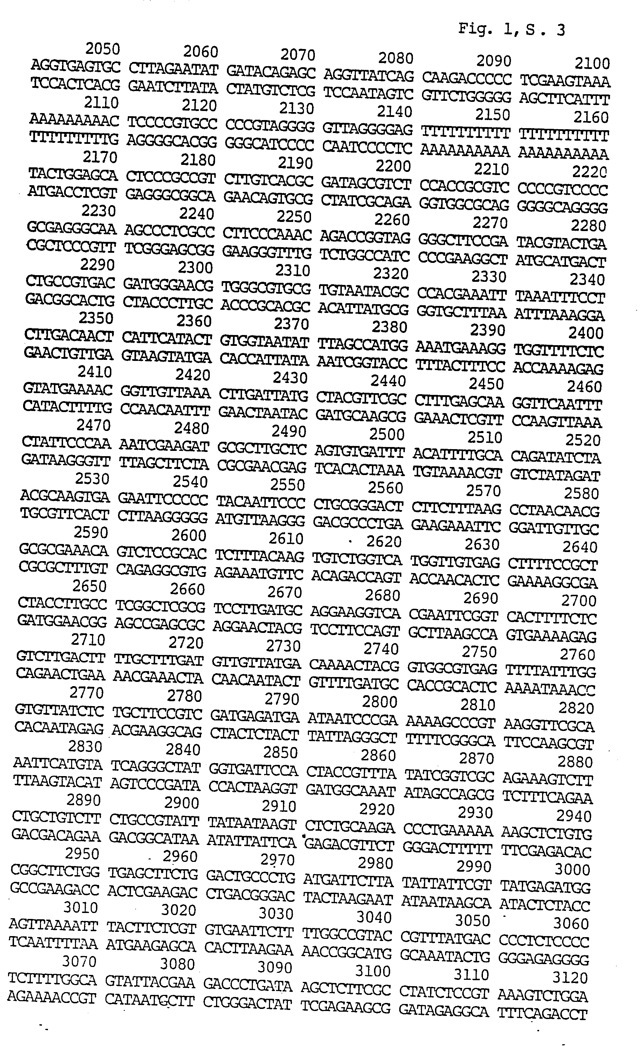

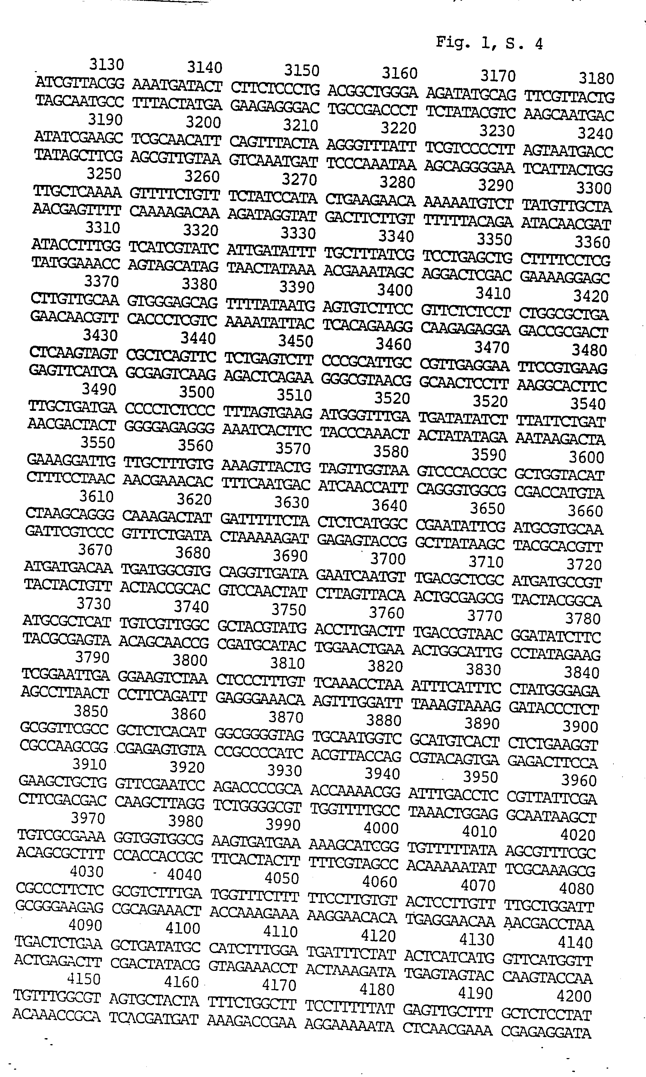

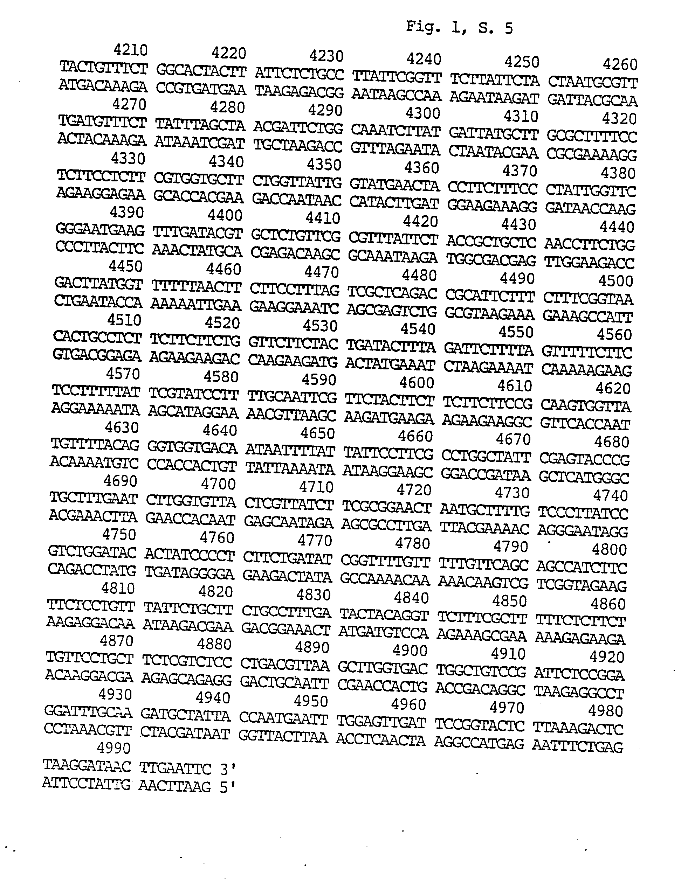

Die Charakterisierung dieser in Fig. 1 gezeigten ursprünglich vorliegenden aus Stuhlisolaten isolierten DNA zeigt, daß es sich um eine partiell doppelsträngige zirkuläre DNA handelt.

Die DNA zeigt Verwandtschaft mit HBV-DNA. Wenn ein kloniertes 0,45 Kb Fragment oder gereinigtes Stuhlmaterial mit $^{32}$P markiert und in einer Southern-Analyse an HBV-DNA hybridisiert wurde, ergaben beide Proben ein Signal, allerdings etwa 1000-fach geringer als mit sich selbst. Plasmid pBR 322 und Lambda Phage ergaben kein Signal. In Vorversuchen konnte die gereingite Stuhlprobe, ohne denaturiert zu werden, sehr gut mit dem kleinen Fragment der E.coli Polymerase markiert werden.

Mit Klenow Polymerase behandelte Stuhlprobe migrierte in einem Agarosegel deutlich langsamer als die unbehandelte Probe. Dies spricht dafür, daß die untersuchte DNA, ebenso wie HBV-DNA, partiell doppelsträngig ist. Die Ergebnisse und Behandlung mit Restriktionsenzymen und Primerextension beweisen eine circuläre Struktur der DNA.

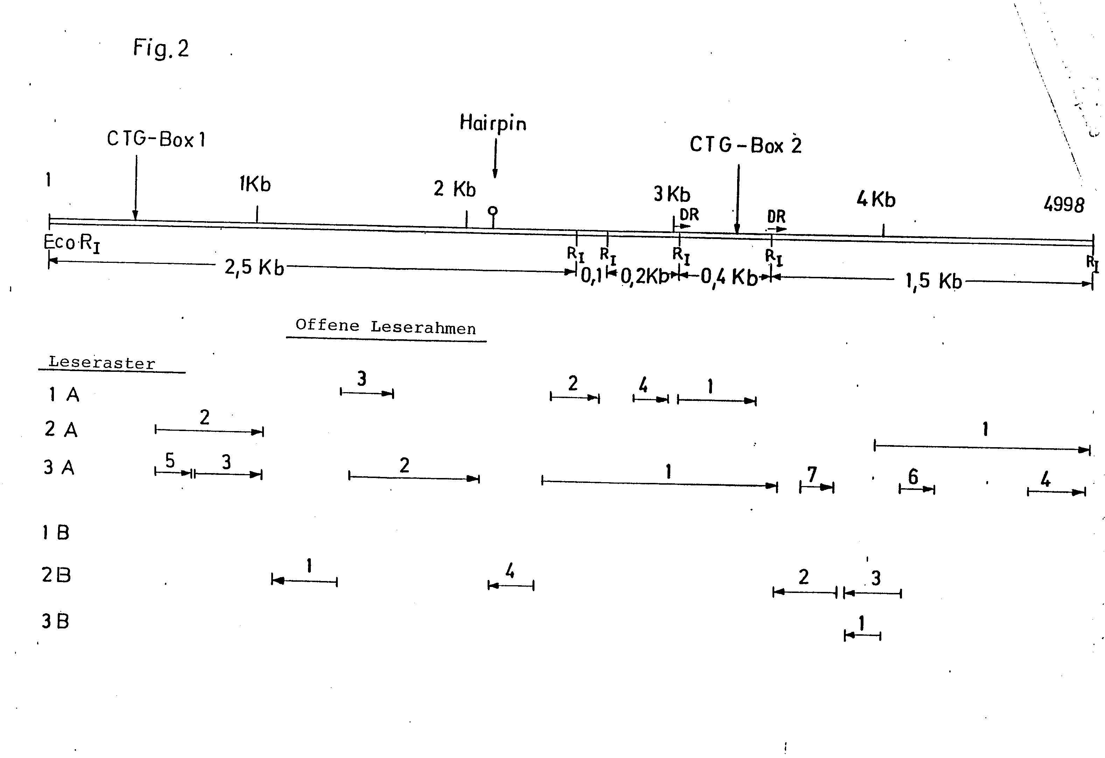

Die mehrfach durchgeführten Sequenzanalysen beider DNA Stränge ergaben 4998 Basenpaare. Die Sequenz ist vom 5'-zum 3'-Ende dargestellt, die Nummerierung mit 1 beginnt im ersten Nucleotid des 2,5 Kb EcoRI-Fragments in dem DNA-Strang, der die großen offenen Leserahmen enthält (siehe Abb. 2). Ein in der Darstellung nicht gezeigtes DNA-Fragment von ca. 10 bis 20 Basenpaaren wurde experimentell nachgewiesen und grenzt an die 2,5 und 1,5 Kb EcoRI-Fragmente und bedingt also den Ringschluß der linear abgebildeten DNA (vgl. Abb.2). Der Nachweis dieses Fragments wurde durch ein Amplifikationsexperiment erbracht, wobei gereinigte Virus-DNA mit Klenow Polymerase und den beiden synthetischen Primern 13 und 17 inkubiert und die DNA-Sequenz zwischen den Primern mehrfach amplifiziert und nach Elektrophorese durch Southern-Analyse nachgewiesen wurde. Da die beiden Primer an den beiden Enden der sequenzierten DNA liegen, kann eine Amplifizierung eines Fragmentes nur erfolgen, falls die DNA zirkulär vorliegt. Das Virus-Genom besitzt damit eine Gesamtlänge von 5.010 bis maximal 5.050 Basenpaare. Dieses Resultat wird zusätzlich durch unabhängige Southern-Analyse des Genoms bestätigt. Die Restriktionskarte des Genoms liegt vor. Die Reihenfolge z.B. der einzelnen EcoRI-Fragmente ist: 1,5 / 0,45 / 0,3 / 0,15 und 2,5 Kb.

Bezüglich besonderer Merkmale der Sequenz ist auszuführen, daß eine lange palindromische Sequenz (Hairpin) zwischen den Basenpaaren 2.097 und 2.149 am Ende eines offenen Leserahmens liegt (Pos. 4, Abb. 2).

An der Position 424 und 3.303 befindet sich je eine "CTG"-Box. Bei der CTG-Box von 3.303 befinden sich auch 2 Repeats, die Sequenzhomologie mit den direkten Repeats der Hepadnaviridae aufweisen.

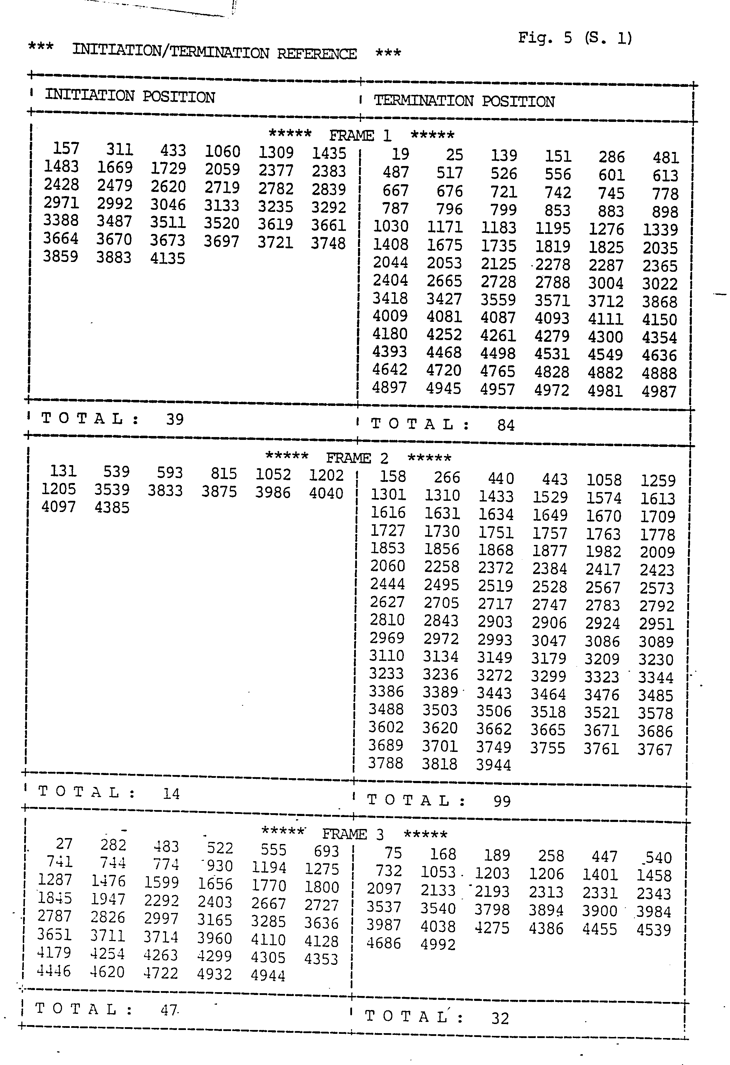

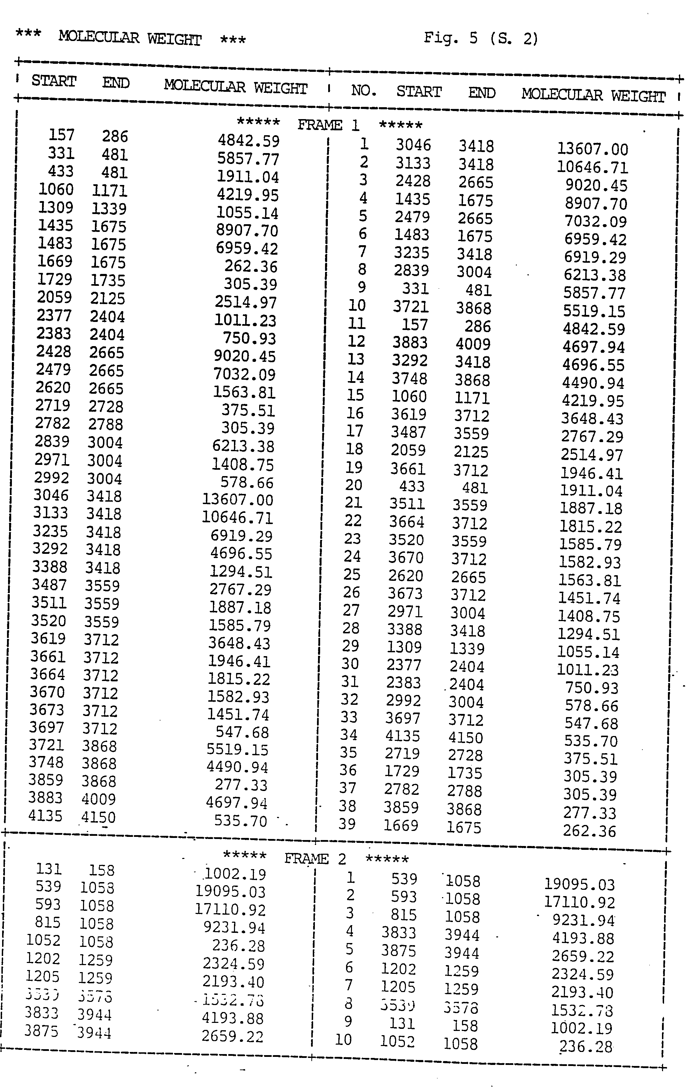

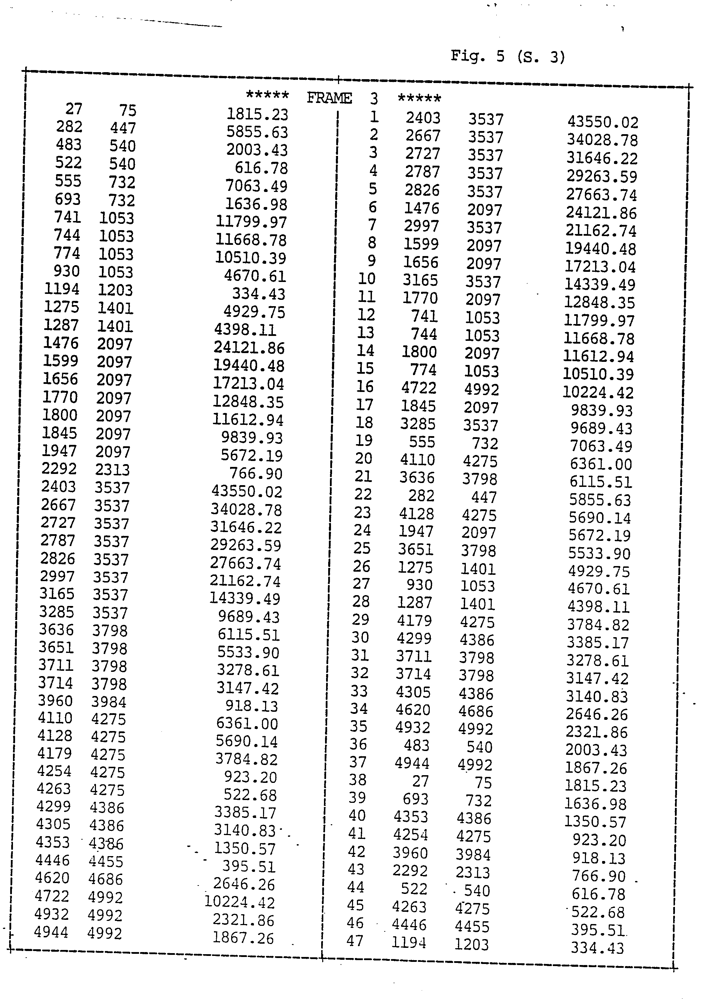

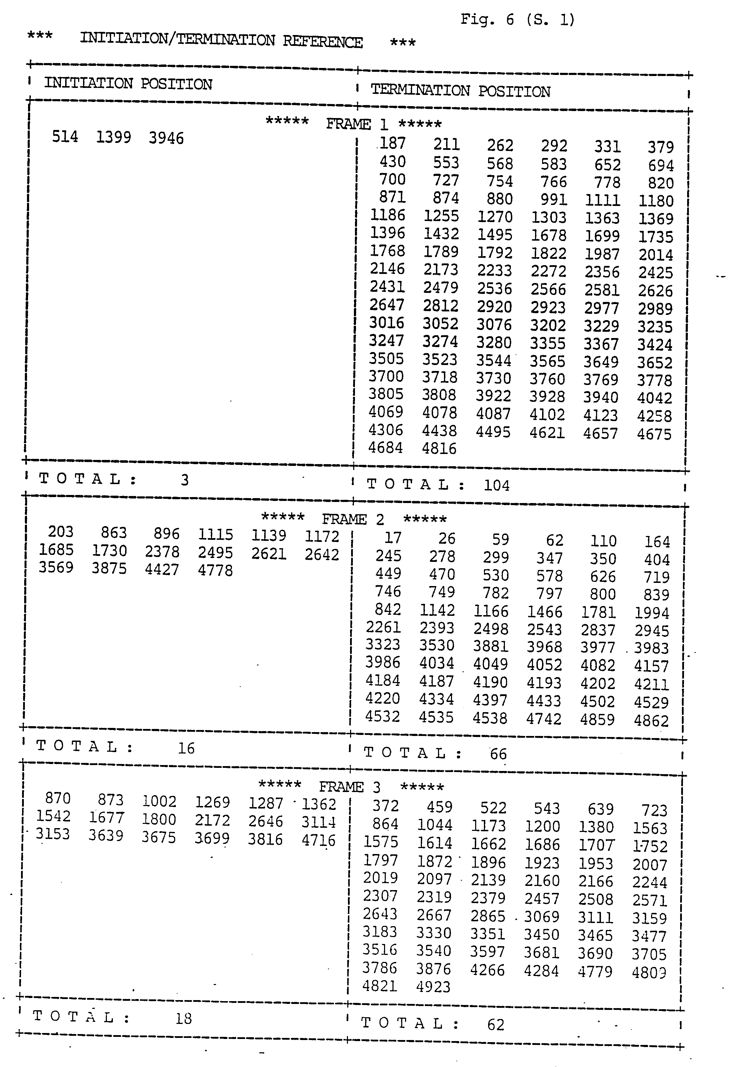

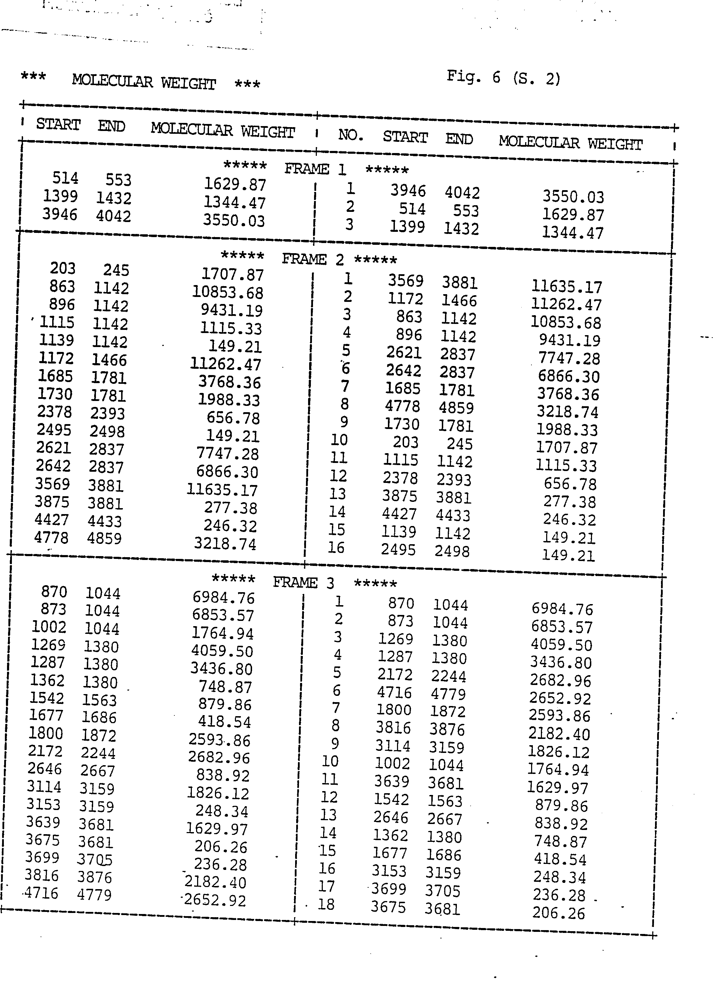

Die offenen Leserahmen finden sich hauptsächlich nur auf einem Strang. Der andere Strang ist wie bei Hepatitis B-Virus-DNA bis auf kleinere Peptide geschlossen. Während der offene DNA-Strang ohne weitere Modifizierung für Proteine bis zu Molekulargewichten über 40.000 codieren kann, sind im Komplementärstrang weite Bereiche aller drei Leseraster geschlossen bis auf 5 Peptide von einem kleineren Molekulargewicht von ca. 6.000 (siehe Abb. 2 und Abb. 5 und 6).

Alle überlappenden Clone ergaben für die gleiche Sequenz identische Resultate mit einer Ausnahme, wo an der Pos. 2.381 in einem Fall G, in einem anderen Fall A gefunden wurde. Ein Sequenzierfehler ist auszuschließen.

Das gibt einen Hinweis darauf, was durch weitere Befunde bestätigt wird, daß Abweichungen von einigen Prozent, insbeondere 1 bis 2 %, in der Regel keinen Einfluß auf die Funktion der Sequenz haben, so daß funktionelle Äquivalente Abweichungen bis 5 %, insbesondere bis 2 % von der Grundstruktur haben können. Das gleiche gilt auch für die von solchen DNAs kodierten Proteinen.

Zusammenfassend läßt sich somit folgendes sagen:

Die Non-A, Non-B-assoziierte Substanz ist gekennzeichnet durch die signifikante Bindung an Immunglobuline und die Bindung an Fab2-Bruchstücke gereinigben IgGs, durch Bindung an nicht kollagenbindende Fibronectinspaltprodukte sowie durch die Infektiösität gegenüber menschlichen Zellkulturlinien, die morphologisch darstellbar sind durch virustypische Veränderungen derselben und molekulargenetisch durch den Nachweis einer spezifisch hybridisierenden, gelelektrophoretisch bei einer scheinbaren Größe von 3,2 KB wandernden DNA (ungeschnitten und unter nativen Bedingungen), innerhalb dieser Zellkulturen.

Die in den infizierten Zellen sowie im Serum von Non-A, Non-B-Hepatitis-Patienten nachweisbare DNA hybridisiert mit der aus Non-A, Non-B-Substanz isolierten, ca. 5 KB großen DNA bzw. der aus diesem Material hergestellten klonierten DNA.

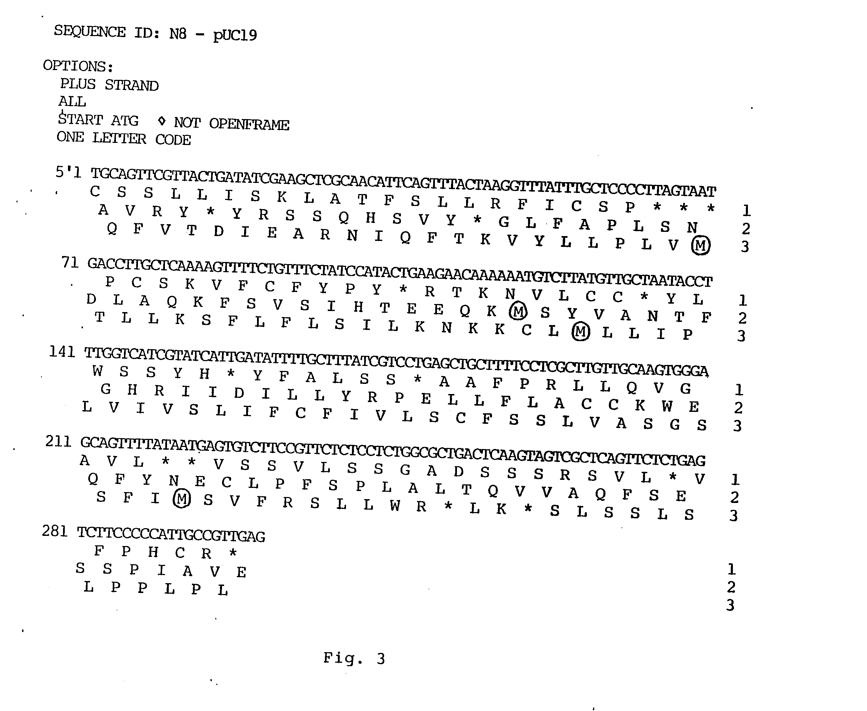

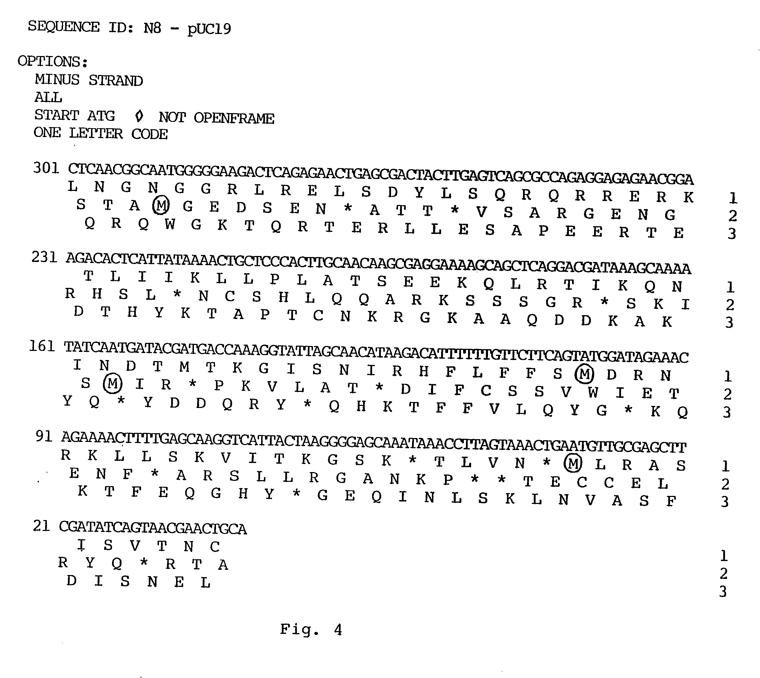

Die gesamte Sequenz von ca. 5.000 Basenpaaren gemäß Fig. 1 und eine Teilsequenz von ca. 300 Basenpaaren gemäß Fig. 3 und 4 der klonierten DNA wurden bestimmt und mit bekannten menschlichen DNA-Sequenzen, Phagen-DNA-Sequenzen, Plasmid-DNA-Sequenzen und bekannten publizierten Virus-DNA-Sequenzen verglichen. Sie entsprechen nach diesen Daten keiner bisher beschriebenen Sequenz. Nach dem offenen Leserahmen der Sequenz kann man Rückschlüsse auf die kodierten und exprimierten virusspezifischen Proteine ziehen. Damit kann man die hydrophilen und hydrophoben Regionen innerhalb der Peptide identifizieren und somit ist die Herstellung synthetischer Peptide aus den möglichen antigenen Epitopen in den hydrophilen Regionen möglich.

Die gefundene DNA, insbesondere die auf dieser DNA sich befindlichen Gene können zur Insertion in entsprechende Expressionsvektoren, wie Zellen und Bakterien (z.B. E.coli und Hefen, wie Syccharomyces cereviciae sowie Zellkulturen), und damit zur Herstellung von Virusantigenen benutzt werden. Damit ist die Diagnostik und die Herstellung von Immunreagentien und Vaccinen möglich. Bei der Diagnostik sind insbesondere die Untersuchung auf Infektiosität (Blutkonservenuntersuchung) und die Diagnose einer akuten, chronischen oder zeitlich zurückliegenden Infektion zu nennen. In Zellkulturen hergestellte Antigene sowie die entsprechenden, durch diese Virus-DNA in vivo und in vitro synthetisierten Proteine können zur Erstellung immunologischer Diagnostika benutzt werden. Die DNA-Sequenz läßt sich zur Identifizierung potentieller Virusproteine und deren synthetischer Herstellung, also die Herstellung synthetischer antigener Peptide, verwenden, ebenso wie sich die DNA bzw. DNA-Teilsequenzen zur Herstellung synthetischer DNA oder RNA bzw. DNA-oder RNA-Fragmente und für den Einsatz derselben als Sonden oder Primer für die Diagnostik einsetzen lassen. Schließlich kann man die DNA bzw. die erstellte DNA-Sequenz zur Herstellung synthetischer Viren (vollkommene DNA-Synthese) und zur Insertion von synthetischer DNA oder DNA-Fragmente in entsprechende Vektoren verwenden, um Virusexpressionsprodukte zu erhalten.

## Ansprüche

1. NANB-Hepatitis assoziierte DNA, enthaltend vor allem die DNA nach Figur 1 sowie funktionelle Äquivalente und Teilsequenzen davon.

2. NANB-Hepatitis assoziierte DNA nach Anspruch 1, dadurch gekennzeichnet, daß sie höchstens 5 %, insbesondere höchstens 2 % Abweichung von der Struktur und/oder der Kettenlänge der DNA nach Fig. 1 zeigt.

3. NANB-Hepatitis assoziierte DNA nach Anspruch 1 oder 2, dadurch gekennzeichnet, daß sie partiell doppelsträngig zirkulär und aus einem NANB-Hepatitis assoziierten Partikel bzw. aus Virus isoliert ist.

4. DNA-Teilsequenz nach Anspruch 1 -3, dadurch gekennzeichnet, daß sie Fig. 3 bzw. 4 mit maximal 5 %, vorzugsweise maximal 2 %,Abweichung entspricht.

5. Proteine, dadurch gekennzeichnet, daß sie durch eine DNA nach Anspruch 1 bis 4 kodiert sind oder derart kodierten Proteinen entsprechen.

6. Proteine nach Anspruch 5, dadurch gekennzeichnet, daß sie den Sequenzen der offenen Leserahmen gemäß Fig. 5 und 6 entsprechen.

7. Verfahren zur Herstellung der NANB-assoziierten DNAs nach Anspruch 1 -4 (sowie der Proteine nach Anspruch 5 und 6) aus Stuhl, Gewebe, Körperflüssigkeiten oder Viruskulturen, dadurch gekennzeichnet, daß das Ausgangsmaterial, ggfs. nach vorheriger Sterilfiltration und Fällung mit PEG bei Stuhlsuspensionen, mit DNase und ggfs. RNase zur Verdauung inkubiert wird, dann ein Dichtegradient, insbesondere CsCl, d = 1,3, angelegt wird, worauf Dialyse, Verdauung mit Proteinase K, Phenolextraktion, Äthanolfällung und ggfs. Extraktion und erneute Fällung und Isolierung sowie ggfs. Klonen und ggfs. exprimieren in entsprechenden Vektoren erfolgen.

8. Verwendung der DNAs nach Anspruch 1 bis 4 als DNA-Sonden zur Diagnose, insbesondere nach klassischen Hybridisierungsverfahren, oder zur Expression in geeigneten Vektoren.

9. Verwendung der DNAs nach Anspruch 1 bis 4 zur Synthese von Proteinen zur Erzeugung immunologischer Reagentien für den Nachweis von NANB-Hepatitis und zur Erzeugung von Impfstoffen.

10. Verwendung der DNAs nach Anspruch 1 bis 4 oder der auf diesen DNAs befindlichen Gene bzw. der entsprechenden DNA-Sequenzen zur Herstellung synthetischer Viren und zur Insertion der natürlichen oder synthetischen DNAs oder DNA-Fragmente in entsprechende Vektoren zur Bildung von Virusexpressionsprodukten und zur Herstellung von Virusantigenen.

0 279 460

FILE: TONGA.DNA     SEQUENCE: 4998BP;     996 A;     1130C;     1087 G;     1

*** SEQUENCE LIST ***                    (DOUBLE)

```
             10         20         30         40         50         60
5' GAATTCTGCC TCTTCTGTTG ATTCTGATGT TGCCTCCGGT GCTTCTGGTC TTCTTGGCGG
3' CTTAAGACGG AGAAGACAAC TAAGACTACA ACGGAGGCCA CGAAGACCAG AAGAACCGCC
             70         80         90        100        110        120
   TCTTTTCCAG AATTTAGGCA CGTTTCTTTT CTCTGTTTCT CTCCTTTGCT TTGGCGCTGT
   AGAAAAGGTC TTAAATCCGT GCAAAGAAAA GAGACAAAGA GAGGAAACGA AACCGCGACA
            130        140        150        160        170        180
   TGTTCTTCGA ATGCTTATTA GAAAGGCGGT TGACGGATGA CTTTTCTTGA TTTCTTCAAA
   ACAAGAAGCT TACGAATAAT CTTTCCGCCA ACTGCCTACT GAAAAGAACT AAAGAAGTTT
            190        200        210        220        230        240
   TCAGTTTTTA ACCTTTTCGG TTCTGGTGGC GCTCTCGTCA TTGCCGTTGT TGTTTTTCTT
   AGTCAAAAAT TGGAAAAGCC AAGACCACCG CGAGAGCAGT AACGGCAACA ACAAAAAGAA
            250        260        270        280        290        300
   GTCGGCCTCG GTATTTATAA GTTCGTAAAG GATTGGTTGC CATGGTAGAC TTTGTTTCCG
   CAGCCGGAGC CATAAATATT CAAGCATTTC CTAACCAACG GTACCATCTG AAACAAAGGC
            310        320        330        340        350        360
   CTCTTGGCGT TTTTACCTCG TTTATCGCCA ATGTGCTTTC TATTTCCTTT TTTGGCTTTG
   GAGAACCGCA AAAATGGAGC AAATAGCGGT TACACGAAAG ATAAAGGAAA AAACCGAAAC
            370        380        390        400        410        420
   GTACTTTTGG CAACTTTATT TTGGTTTGTC TTTTGCTTTC GCTTGTTGGC TTTGTTCTCT
   CATGAAAACC GTTGAAATAA AACCAAACAG AAAACGAAAG CGAACAACCG AAACAAGAGA
            430        440        450        460        470        480
   GTGGCCTTTG GGATGGAGGT GATAAATAGT GGAAATCCCT ATTATTATCA ATACTTGGGT
   CACCGGAAAC CCTACCTCCA CTATTTATCA CCTTTAGGGA TAATAATAGT TATGAACCCA
            490        500        510        520        530        540
   TGATGCTGAC GGCGTTACCG TCTATACAGT GCAGTATAAA GATGGTAGCA CTTGCGATAT
   ACTACGACTG CCGCAATGGC AGATATGTCA CGTCATATTT CTACCATCGT GAACGCTATA
            550        560        570        580        590        600
   GACCGTCCAG CAGTATGATT ATCTCAAGGC ATCCGCGCAG GCTGTCGCCG ATATGGACTC
   CTGGCAGGTC GTCATACTAA TAGAGTTCCG TAGGCGCGTC CGACAGCGGC TATACCTGAG
            610        620        630        640        650        660
   TAAAGCCGCT GCTGATTCTC CTTCGGAAGC TGCTCCTGCT CCCGAGGAAC CTGCACAGAA
   ATTTCGGCGA CGACTAAGAG GAAGCCTTCG ACGAGGACGA GGGCTCCTTG GACGTGTCTT
            670        680        690        700        710        720
   TATTATTGAA TCTCCTGACC TCCGCGAAGG TTATGTGCCG CAGGAAGAAG AATTACCTTT
   ATAATAACTT AGAGGACTGG AGGCGCTTCC AATACACGGC GTCCTTCTTC TTAATGGAAA
            730        740        750        760        770        780
   TGAGGGAGT TTAACCGCTT ATGATGACCG CGCAGCAGAT ACTCCGGCTT TGTATGCTAA
   ACTCCCCTCA AATTGGCGAA TACTACTGGC GCGTCGTCTA TGAGGCCGAA ACATACGATT
            790        800        810        820        830        840
   TCTCCCTAAC GTCTCTAATA GTTCACTAC TATTATGGAT TGGTTCGGAG ATACGTTTTT
   AGAGGGATTG CAGAGATTAT CAAAGTGATG ATAATACCTA ACCAAGCCTC TATGCAAAAA
            850        860        870        880        890        900
   TATCGAACGT ACTGAGACGG TGCACAAGTC CGGCTATACG TCTGAAAGGT ATTCCTATAA
   ATAGCTTGCA TGACTCTGCC ACGTGTTCAG GCCGATATGC AGACTTTCCA TAAGGATATT
            910        920        930        940        950        960
   CAGCTCGACT CAACTTATTC AGCTTCCTTA TGCGGAGGAT TCCACTACTA CGTCTCAGGT
   GTCGAGCTGA GTTGAATAAG TCGAAGGAAT ACGCCTCCTA AGGTGATGAT GCAGAGTCCA
```

Fig. 1, S. 2

```
          970        980        990       1000       1010       1020
TCTCAATCCG CAAGCTTGCG TTTCTGCTTT GCTTGTTGTC CTTGTCTTCG TTACTACTGT
AGAGTTAGGC GTTCGAACGC AAAGACGAAA CGAACAACAG GAACAGAAGC AATGATGACA
         1030       1040       1050       1060       1070       1080
TACTTGGATT AAAAACGCGA TTTGGGGGCG CATGAGTTAA TGGAAATTCT ACCTTTACAG
ATGAACCTAA TTTTTGCGCT AAACCCCCGC GTACTCAATT ACCTTTAAGA TGGAAATGTC
         1090       1100       1110       1120       1130       1140
TATTGTTTCG GTATCTTCTC TGTCCCCGAA ATTGGCTATT TCATTGTCTT CGCTGCTGTT
ATAACAAAGC CATAGAAGAG ACAGGGGCTT TAACCGATAA AGTAACAGAA GCGACGACAA
         1150       1160       1170       1180       1190       1200
TTCTCTTTGT TGGTCCTCCT GCTCCGTCCG TGACAGGTGC CATAAATATT ATTATGAAAG
AAGAGAAACA ACCAGGAGGA CGAGGCAGGC ACTGTCCACG GTATTTATAA TAATACTTTC
         1210       1220       1230       1240       1250       1260
GATGATGACT TCAGGGCGCT ACTTCTTCTA TTCTCGCTAC GCTGCTTTCC TTGGTCGGTG
CTACTACTGA AGTCCCGCGA TGAAGAAGAT AAGAGCGATG CGACGAAAGG AACCAGCCAC
         1270       1280       1290       1300       1310       1320
AGTTCTTTAC CTCGATGATT ACTTGGATGG GTCAGCTCAT TGATTTCTAT GAGTCTCAGC
TCAAGAAATG GAGCTACTAA TGAACCTACC CAGTCGAGTA ACTAAAGATA CTCAGAGTCG
         1330       1340       1350       1360       1370       1380
CCATTCTCCT TGTCTTCGTG ATTCTCACTA TCGCGGGCAT TGTTCTCCGT ATCCTTCGCC
GGTAAGAGGA ACAGAAGCAC TAAGAGTGAT AGCGCCCGTA ACAAGAGGCA TAGGAAGCGG
         1390       1400       1410       1420       1430       1440
GCTGGATTCC TGGTCGCTCC TAACGACTGA GAGAAACGC CGCCGACCAT TTTAATGGTC
CGACCTAAGG ACCAGCGAGG ATTGCTGACT CTCTTTTGCG GCGGCTGGTA AAATTACCAG
         1450       1460       1470       1480       1490       1500
GGCGACGTTT TCTTATTTAG AAAGGATTAT TTGTTATGCT TTATGGTATT CTTATCTTTT
CCGCTGCAAA AGAATAAATC TTTCCTAATA AACAATACGA AATACCATAA GAATAGAAAA
         1510       1520       1530       1540       1550       1560
GCGTTTGCTG GCTTTTTGTT TATATCGATA ACTATTGCAA AAATCCCTAC AAGCTCGAAG
CGCAAACGAC CGAAAAACAA ATATAGCTAT TGATAACGTT TTTAGGGATG TTCGAGCTTC
         1570       1580       1590       1600       1610       1620
CTGTTGTTGG TTCTAAAGGT TCTGGCAAGT CTCTGTATAT GTCTCGCGTT GCTGATAAGT
GACAACAACC AAGATTTCCA AGACCGTTCA GAGACATATA CAGAGCGCAA CGACTATTCA
         1630       1640       1650       1660       1670       1680
GGCTTCGTTC TAGTAAGGGG TTTATTTATA GCAATATGGG TATTGGTTAT GATTTAGAGC
CCGAAGCAAG ATCATTCCCC AAATAAATAT CGTTATACCC ATAACCAATA CTAAATCTCG
         1690       1700       1710       1720       1730       1740
CGGAATATTG GAAACAGACC TTTGCCCCTG ATTCCCTTAT TCTTATTGAT GAGATAGGCG
GCCTTATAAC CTTTGTCTGG AAACGGGGAC TAAGGGAATA AGAATAACTA CTCTATCCGC
         1750       1760       1770       1780       1790       1800
TGCTCCACTC TAACCGTGAT TTTAAGGCTA TGCCCCGTGA AGCTGTCGAG TTTTTCAAGA
ACGAGGTGAG ATTGGCACTA AAATTCCGAT ACGGGGCACT TCGACAGCTC AAAAAGTTCT
         1810       1820       1830       1840       1850       1860
TGCAGCGCAA ATATCACTTG ACAATAGTTG TATCGTCTCA GACCATGGAC TTTGATAAAA
ACGTCGCGTT TATAGTGAAC TGTTATCAAC ATAGCAGAGT CTGGTACCTG AAACTATTTT
         1870       1880       1890       1900       1910       1920
AGATTCGTGA CCTCTGTGAT CGCATTTATC TCTGCAATCG TATTGGCTGG TTTTGTCGCC
TCTAAGCACT GGAGACACTA GCGTAAATAG AGACGTTAGC ATAACCGACC AAAACAGCGG
         1930       1940       1950       1960       1970       1980
TCACCCCTTA TCGCTCCTGT ATCGCTATGG AACACCGTCC CGAGGGCGGC CAAGAGCTTG
AGTGGGGAAT AGCGAGGACA TAGCGATACC TTGTGGCAGG GCTCCCGCCG GTTCTCGAAC
         1990       2000       2010       2020       2030       2040
TTAACACGGT GCGCAAGGCG GGCAGGGCTA AGTGGTATAC TATCCCCAAG TCTGTGAAGC
AATTGTGCCA CGCGTTCCGC CCGTCCCGAT TCACCATATG ATAGGGGTTC AGACACTTCG
```

0 279 460

Fig. 1, S. 3

```
        2050       2060       2070       2080       2090       2100
AGGTGAGTGC CTTAGAATAT GATACAGAGC AGGTTATCAG CAAGACCCCC TCGAAGTAAA
TCCACTCACG GAATCTTATA CTATGTCTCG TCCAATAGTC GTTCTGGGGG AGCTTCATTT
        2110       2120       2130       2140       2150       2160
AAAAAAAAAC TCCCCGTGCC CCCGTAGGGG GTTAGGGGAG TTTTTTTTTT TTTTTTTTTT
TTTTTTTTTG AGGGGCACGG GGGCATCCCC CAATCCCCTC AAAAAAAAAA AAAAAAAAAA
        2170       2180       2190       2200       2210       2220
TACTGGAGCA CTCCCGCCGT CTTGTCACGC GATAGCGTCT CCACCGCGTC CCCCGTCCCC
ATGACCTCGT GAGGGCGGCA GAACAGTGCG CTATCGCAGA GGTGGCGCAG GGGGCAGGGG
        2230       2240       2250       2260       2270       2280
GCGAGGGCAA AGCCCTCGCC CTTCCCAAAC AGACCGGTAG GGGCTTCCGA TACGTACTGA
CGCTCCCGTT TCGGGAGCGG GAAGGGTTTG TCTGGCCATC CCCGAAGGCT ATGCATGACT
        2290       2300       2310       2320       2330       2340
CTGCCGTGAC GATGGGAACG TGGGCGTGCG TGTAATACGC CCACGAAATT TAAATTTCCT
GACGGCACTG CTACCCTTGC ACCCGCACGC ACATTATGCG GGTGCTTTAA ATTTAAAGGA
        2350       2360       2370       2380       2390       2400
CTTGACAACT CATTCATACT GTGGTAATAT TTAGCCATGG AAATGAAAGG TGGTTTTCTC
GAACTGTTGA GTAAGTATGA CACCATTATA AATCGGTACC TTTACTTTCC ACCAAAAGAG
        2410       2420       2430       2440       2450       2460
GTATGAAAAC GGTTGTTAAA CTTGATTATG CTACGTTCGC CTTTGAGCAA GGTTCAATTT
CATACTTTTG CCAACAATTT GAACTAATAC GATGCAAGCG GAAACTCGTT CCAAGTTAAA
        2470       2480       2490       2500       2510       2520
CTATTCCCAA AATCGAAGAT GCGCTTGCTC AGTGTGATTT ACATTTTGCA CAGATATCTA
GATAAGGGTT TTAGCTTCTA CGCGAACGAG TCACACTAAA TGTAAAACGT GTCTATAGAT
        2530       2540       2550       2560       2570       2580
ACGCAAGTGA GAATTCCCCC TACAATTCCC CTGCGGGACT CTTCTTTAAG CCTAACAACG
TGCGTTCACT CTTAAGGGGG ATGTTAAGGG GACGCCCTGA GAAGAAATTC GGATTGTTGC
        2590       2600       2610       2620       2630       2640
GCGCGAAACA GTCTCCGCAC TCTTTACAAG TGTCTGGTCA TGGTTGTGAG CTTTTCCGCT
CGCGCTTTGT CAGAGGCGTG AGAAATGTTC ACAGACCAGT ACCAACACTC GAAAAGGCGA
        2650       2660       2670       2680       2690       2700
CTACCTTGCC TCGGCTCGCG TCCTTGATGC AGGAAGGTCA CGAATTCGGT CACTTTTCTC
GATGGAACGG AGCCGAGCGC AGGAACTACG TCCTTCCAGT GCTTAAGCCA GTGAAAAGAG
        2710       2720       2730       2740       2750       2760
GTCTTGACTT TTGCTTTGAT GTTGTTATGA CAAAACTACG GTGGCGTGAG TTTTATTTGG
CAGAACTGAA AACGAAACTA CAACAATACT GTTTTGATGC CACCGCACTC AAAATAAACC
        2770       2780       2790       2800       2810       2820
GTGTTATCTC TGCTTCCGTC GATGAGATGA ATAATCCCGA AAAAGCCCGT AAGGTTCGCA
CACAATAGAG ACGAAGGCAG CTACTCTACT TATTAGGGCT TTTTCGGGCA TTCCAAGCGT
        2830       2840       2850       2860       2870       2880
AATTCATGTA TCAGGGCTAT GGTGATTCCA CTACCGTTTA TATCGGTCGC AGAAAGTCTT
TTAAGTACAT AGTCCCGATA CCACTAAGGT GATGGCAAAT ATAGCCAGCG TCTTTCAGAA
        2890       2900       2910       2920       2930       2940
CTGCTGTCTT CTGCCGTATT TATAATAAGT CTCTGCAAGA CCCTGAAAAA AAGCTCTGTG
GACGACAGAA GACGGCATAA ATATTATTCA GAGACGTTCT GGGACTTTTT TTCGAGACAC
        2950       2960       2970       2980       2990       3000
CGGCTTCTGG TGAGCTTCTG GACTGCCCTG ATGATTCTTA TATTATTCGT TATGAGATGG
GCCGAAGACC ACTCGAAGAC CTGACGGGAC TACTAAGAAT ATAATAAGCA ATACTCTACC
        3010       3020       3030       3040       3050       3060
AGTTAAAATT TACTTCTCGT GTGAATTCTT TTGGCCGTAC CGTTTATGAC CCCTCTCCCC
TCAATTTTAA ATGAAGAGCA CACTTAAGAA AACCGGCATG GCAAATACTG GGGAGAGGGG
        3070       3080       3090       3100       3110       3120
TCTTTTGGCA GTATTACGAA GACCCTGATA AGCTCTTCGC CTATCTCCGT AAAGTCTGGA
AGAAAACCGT CATAATGCTT CTGGGACTAT TCGAGAAGCG GATAGAGGCA TTTCAGACCT
```

```
        3130        3140        3150        3160        3170        3180
     ATCGTTACGG  AAATGATACT  CTTCTCCCTG  ACGGCTGGGA  AGATATGCAG  TTCGTTACTG
     TAGCAATGCC  TTTACTATGA  GAAGAGGGAC  TGCCGACCCT  TCTATACGTC  AAGCAATGAC
        3190        3200        3210        3220        3230        3240
     ATATCGAAGC  TCGCAACATT  CAGTTTACTA  AGGGTTTATT  TCGTCCCCTT  AGTAATGACC
     TATAGCTTCG  AGCGTTGTAA  GTCAAATGAT  TCCCAAATAA  AGCAGGGAA  TCATTACTGG
        3250        3260        3270        3280        3290        3300
     TTGCTCAAAA  GTTTTCTGTT  TCTATCCATA  CTGAAGAACA  AAAAATGTCT  TATGTTGCTA
     AACGAGTTTT  CAAAAGACAA  AGATAGGTAT  GACTTCTTGT  TTTTTACAGA  ATACAACGAT
        3310        3320        3330        3340        3350        3360
     ATACCTTTGG  TCATCGTATC  ATTGATATTT  TGCTTTATCG  TCCTGAGCTG  CTTTTCCTCG
     TATGGAAACC  AGTAGCATAG  TAACTATAAA  ACGAAATAGC  AGGACTCGAC  GAAAAGGAGC
        3370        3380        3390        3400        3410        3420
     CTTGTTGCAA  GTGGGAGCAG  TTTTATAATG  AGTGTCTTCC  GTTCTCTCCT  CTGGCGCTGA
     GAACAACGTT  CACCCTCGTC  AAAATATTAC  TCACAGAAGG  CAAGAGAGGA  GACCGCGACT
        3430        3440        3450        3460        3470        3480
     CTCAAGTAGT  CGCTCAGTTC  TCTGAGTCTT  CCCGCATTGC  CGTTGAGGAA  TTCCGTGAAG
     GAGTTCATCA  GCGAGTCAAG  AGACTCAGAA  GGGCGTAACG  GCAACTCCTT  AAGGCACTTC
        3490        3500        3510        3520        3520        3540
     TTGCTGATGA  CCCCTCTCCC  TTTAGTGAAG  ATGGGTTTGA  TGATATATCT  TTATTCTGAT
     AACGACTACT  GGGGAGAGGG  AAATCACTTC  TACCCAAACT  ACTATATAGA  AATAAGACTA
        3550        3560        3570        3580        3590        3600
     GAAAGGATTG  TTGCTTTGTG  AAAGTTACTG  TAGTTGGTAA  GTCCCACCGC  GCTGGTACAT
     CTTTCCTAAC  AACGAAACAC  TTTCAATGAC  ATCAACCATT  CAGGGTGGCG  CGACCATGTA
        3610        3620        3630        3640        3650        3660
     CTAAGCAGGG  CAAAGACTAT  GATTTTTCTA  CTCTCATGGC  CGAATATTCG  ATGCGTGCAA
     GATTCGTCCC  GTTTCTGATA  CTAAAAAGAT  GAGAGTACCG  GCTTATAAGC  TACGCACGTT
        3670        3680        3690        3700        3710        3720
     ATGATGACAA  TGATGGCGTG  CAGGTTGATA  GAATCAATGT  TGACGCTCGC  ATGATGCCGT
     TACTACTGTT  ACTACCGCAC  GTCCAACTAT  CTTAGTTACA  ACTGCGAGCG  TACTACGGCA
        3730        3740        3750        3760        3770        3780
     ATGCGCTCAT  TGTCGTTGGC  GCTACGTATG  ACCTTGACTT  TGACCGTAAC  GGATATCTTC
     TACGCGAGTA  ACAGCAACCG  CGATGCATAC  TGGAACTGAA  ACTGGCATTG  CCTATAGAAG
        3790        3800        3810        3820        3830        3840
     TCGGAATTGA  GGAAGTCTAA  CTCCCTTTGT  TCAAACCTAA  ATTTCATTTC  CTATGGGAGA
     AGCCTTAACT  CCTTCAGATT  GAGGGAAACA  AGTTTGGATT  TAAAGTAAAG  GATACCCTCT
        3850        3860        3870        3880        3890        3900
     GCGGTTCGCC  GCTCTCACAT  GGCGGGGTAG  TGCAATGGTC  GCATGTCACT  CTCTGAAGGT
     CGCCAAGCGG  CGAGAGTGTA  CCGCCCCATC  ACGTTACCAG  CGTACAGTGA  GAGACTTCCA
        3910        3920        3930        3940        3950        3960
     GAAGCTGCTG  GTTCGAATCC  AGACCCCGCA  ACCAAAACGG  ATTTGACCTC  CGTTATTCGA
     CTTCGACGAC  CAAGCTTAGG  TCTGGGGCGT  TGGTTTTGCC  TAAACTGGAG  GCAATAAGCT
        3970        3980        3990        4000        4010        4020
     TGTCGCGAAA  GGTGGTGGCG  AAGTGATGAA  AAAGCATCGG  TGTTTTTATA  AGCGTTTCGC
     ACAGCGCTTT  CCACCACCGC  TTCACTACTT  TTTCGTAGCC  ACAAAAATAT  TCGCAAAGCG
        4030        4040        4050        4060        4070        4080
     CGCCCTTCTC  GCGTCTTTGA  TGGTTTCTTT  TTCCTTGTGT  ACTCCTTGTT  TTGCTGGATT
     GCGGGAAGAG  CGCAGAAACT  ACCAAAGAAA  AAGGAACACA  TGAGGAACAA  AACGACCTAA
        4090        4100        4110        4120        4130        4140
     TGACTCTGAA  GCTGATATGC  CATCTTTGGA  TGATTTCTAT  ACTCATCATG  GTTCATGGTT
     ACTGAGACTT  CGACTATACG  GTAGAAACCT  ACTAAAGATA  TGAGTAGTAC  CAAGTACCAA
        4150        4160        4170        4180        4190        4200
     TGTTTGGCGT  AGTGCTACTA  TTTCTGGCTT  TCCTTTTTAT  GAGTTGCTTT  GCTCTCCTAT
     ACAAACCGCA  TCACGATGAT  AAAGACCGAA  AGGAAAAATA  CTCAACGAAA  CGAGAGGATA
```

Fig. 1, S. 5

```
           4210       4220       4230       4240       4250       4260
     TACTGTTTCT GGCACTACTT ATTCTCTGCC TTATTCGGTT TCTTATTCTA CTAATGCGTT
     ATGACAAAGA CCGTGATGAA TAAGAGACGG AATAAGCCAA AGAATAAGAT GATTACGCAA
           4270       4280       4290       4300       4310       4320
     TGATGTTTCT TATTTAGCTA ACGATTCTGG CAAATCTTAT GATTATGCTT GCGCTTTTCC
     ACTACAAAGA ATAAATCGAT TGCTAAGACC GTTTAGAATA CTAATACGAA CGCGAAAAGG
           4330       4340       4350       4360       4370       4380
     TCTTCCTCTT CGTGGTGCTT CTGGTTATTG GTATGAACTA CCTTCTTTCC CTATTGGTTC
     AGAAGGAGAA GCACCACGAA GACCAATAAC CATACTTGAT GGAAGAAAGG GATAACCAAG
           4390       4400       4410       4420       4430       4440
     GGGAATGAAG TTTGATACGT GCTCTGTTCG CGTTTATTCT ACCGCTGCTC AACCTTCTGG
     CCCTTACTTC AAACTATGCA CGAGACAAGC GCAAATAAGA TGGCGACGAG TTGGAAGACC
           4450       4460       4470       4480       4490       4500
     GACTTATGGT TTTTTAACTT CTTCCTTTAG TCGCTCAGAC CGCATTCTTT CTTTCGGTAA
     CTGAATACCA AAAAATTGAA GAAGGAAATC AGCGAGTCTG GCGTAAGAAA GAAAGCCATT
           4510       4520       4530       4540       4550       4560
     CACTGCCTCT TCTTCTTCTG GTTCTTCTAC TGATACTTTA GATTCTTTTA GTTTTTCTTC
     GTGACGGAGA AGAAGAAGAC CAAGAAGATG ACTATGAAAT CTAAGAAAAT CAAAAAGAAG
           4570       4580       4590       4600       4610       4620
     TCCTTTTTAT TCGTATCCTT TTGCAATTCG TTCTACTTCT TCTTCTTCCG CAAGTGGTTA
     AGGAAAAATA AGCATAGGAA AACGTTAAGC AAGATGAAGA AGAAGAAGGC GTTCACCAAT
           4630       4640       4650       4660       4670       4680
     TGTTTTACAG GGTGGTGACA ATAATTTTAT TATTCCTTCG CCTGGCTATT CGAGTACCCG
     ACAAAATGTC CCACCACTGT TATTAAAATA ATAAGGAAGC GGACCGATAA GCTCATGGGC
           4690       4700       4710       4720       4730       4740
     TGCTTTGAAT CTTGGTGTTA CTCGTTATCT TCGCGGAACT AATGCTTTTG TCCCTTATCC
     ACGAAACTTA GAACCACAAT GAGCAATAGA AGCGCCTTGA TTACGAAAAC AGGGAATAGG
           4750       4760       4770       4780       4790       4800
     GTCTGGATAC ACTATCCCCT CTTCTGATAT CGGTTTTGTT TTTGTTCAGC AGCCATCTTC
     CAGACCTATG TGATAGGGGA GAAGACTATA GCCAAAACAA AAACAAGTCG TCGGTAGAAG
           4810       4820       4830       4840       4850       4860
     TTCTCCTGTT TATTCTGCTT CTGCCTTTGA TACTACAGGT TCTTTCGCTT TTTCTCTTCT
     AAGAGGACAA ATAAGACGAA GACGGAAACT ATGATGTCCA AGAAAGCGAA AAAGAGAAGA
           4870       4880       4890       4900       4910       4920
     TGTTCCTGCT TCTCGTCTCC CTGACGTTAA GCTTGGTGAC TGGCTGTCCG ATTCTCCGGA
     ACAAGGACGA AGAGCAGAGG GACTGCAATT CGAACCACTG ACCGACAGGC TAAGAGGCCT
           4930       4940       4950       4960       4970       4980
     GGATTTGCAA GATGCTATTA CCAATGAATT TGGAGTTGAT TCCGGTACTC TTAAAGACTC
     CCTAAACGTT CTACGATAAT GGTTACTTAA ACCTCAACTA AGGCCATGAG AATTTCTGAG
           4990
     TAAGGATAAC TTGAATTC 3'
     ATTCCTATTG AACTTAAG 5'
```

Fig. 2

0 279 460

SEQUENCE ID: N8 - pUC19

OPTIONS:
 PLUS STRAND
 ALL
 START ATG  ◊ NOT OPENFRAME
 ONE LETTER CODE

5'1 TGCAGTTCGTTACTGATATCGAAGCTCGCAACATTCAGTTTACTAAGGTTTATTTGCTCCCCTTAGTAAT
```
   C S S L L I S K L A T F S L L R F I C S P * * *
   A V R Y * Y R S S Q H S V Y * G L F A P L S N
   Q F V T D I E A R N I Q F T K V Y L L P L V ⓜ
```

71 GACCTTGCTCAAAAGTTTTCTGTTTCTATCCATACTGAAGAACAAAAAATGTCTTATGTTGCTAATACCT
```
   P C S K V F C F Y P Y * R T K N V L C C * Y L
   D L A Q K F S V S I H T E E Q K ⓜ S Y V A N T F
   T L L K S F L F L S I L K N K K C L ⓜ L L I P
```

141 TTGGTCATCGTATCATTGATATTTTTGCTTTATCGTCCTGAGCTGCTTTTCCTCGCTTGTTGCAAGTGGGA
```
   W S S Y H * Y F A L S S * A A F P R L L Q V G
   G H R I I D I L L Y R P E L L F L A C C K W E
   L V I V S L I F C F I V L S C F S S L V A S G S
```

211 GCAGTTTTATAATGAGTGTCTTCCGTTCTCTCCTCTGGCGCTGACTCAAGTAGTCGCTCAGTTCTCTGAG
```
   A V L * * V S S V L S S G A D S S S R S V L * V
   Q F Y N E C L P F S P L A L T Q V V A Q F S E
   S F I ⓜ S V F R S L L W R * L K * S L S S L S
```

281 TCTTCCCCCATTGCCGTTGAG
```
   F P H C R *
   S S P I A V E
   L P P L P L
```

Fig. 3

SEQUENCE ID: N8 – pUC19

OPTIONS:
 MINUS STRAND
 ALL
 START ATG  ◊ NOT OPENFRAME
 ONE LETTER CODE

301 CTCAACGGCAATGGGGGAAGACTCAGAGAACTGAGCGACTACTTGAGTCAGCGCCAGAGGAGAGAACGGA
    L  N  G  N  G  G  R  L  R  E  L  S  D  Y  L  S  Q  R  Q  R  R  E  R  K
     S  T  A Ⓜ G  E  D  S  E  N  *  A  T  T  *  V  S  A  R  G  E  N  G
      Q  R  Q  W  G  K  T  Q  R  T  E  R  L  L  E  S  A  P  E  E  R  T  E

231 AGACACTCATTATAAAACTGCTCCCACTTGCAACAAGCGAGGAAAAGCAGCTCAGGACGATAAAGCAAAA
     T  L  I  I  K  L  L  P  L  A  T  S  E  E  K  Q  L  R  T  I  K  Q  N
    R  H  S  L  *  N  C  S  H  L  Q  Q  A  R  K  S  S  S  G  R  *  S  K  I
     D  T  H  Y  K  T  A  P  T  C  N  K  R  G  K  A  A  Q  D  D  K  A  K

161 TATCAATGATACGATGACCAAAGGTATTAGCAACATAAGACATTTTTTGTTCTTCAGTATGGATAGAAAC
     I  N  D  T  M  T  K  G  I  S  N  I  R  H  F  L  F  F  S Ⓜ D  R  N
      S Ⓜ I  R  *  P  K  V  L  A  T  *  D  I  F  C  S  S  V  W  I  E  T
     Y  Q  *  Y  D  D  Q  R  Y  *  Q  H  K  T  F  F  V  L  Q  Y  G  *  K  Q

 91 AGAAAACTTTTTGAGCAAGGTCATTACTAAGGGGAGCAAATAAACCTTAGTAAACTGAATGTTGCGAGCTT
    R  K  L  L  S  K  V  I  T  K  G  S  K  *  T  L  V  N  * Ⓜ L  R  A  S
     E  N  F  *  A  R  S  L  L  R  G  A  N  K  P  *  *  T  E  C  C  E  L
      K  T  F  E  Q  G  H  Y  *  G  E  Q  I  N  L  S  K  L  N  V  A  S  F

 21 CGATATCAGTAACGAACTGCA
     I  S  V  T  N  C
    R  Y  Q  *  R  T  A
     D  I  S  N  E  L

Fig. 4

Fig. 5 (S. 1)

*** INITIATION/TERMINATION REFERENCE ***

| INITIATION POSITION | | | | | | TERMINATION POSITION | | | | | |
|---|---|---|---|---|---|---|---|---|---|---|---|
| \*\*\*\*\* FRAME 1 \*\*\*\*\* | | | | | | | | | | | |
| 157 | 311 | 433 | 1060 | 1309 | 1435 | 19 | 25 | 139 | 151 | 286 | 481 |
| 1483 | 1669 | 1729 | 2059 | 2377 | 2383 | 487 | 517 | 526 | 556 | 601 | 613 |
| 2428 | 2479 | 2620 | 2719 | 2782 | 2839 | 667 | 676 | 721 | 742 | 745 | 778 |
| 2971 | 2992 | 3046 | 3133 | 3235 | 3292 | 787 | 796 | 799 | 853 | 883 | 898 |
| 3388 | 3487 | 3511 | 3520 | 3619 | 3661 | 1030 | 1171 | 1183 | 1195 | 1276 | 1339 |
| 3664 | 3670 | 3673 | 3697 | 3721 | 3748 | 1408 | 1675 | 1735 | 1819 | 1825 | 2035 |
| 3859 | 3883 | 4135 | | | | 2044 | 2053 | 2125 | 2278 | 2287 | 2365 |
| | | | | | | 2404 | 2665 | 2728 | 2788 | 3004 | 3022 |
| | | | | | | 3418 | 3427 | 3559 | 3571 | 3712 | 3868 |
| | | | | | | 4009 | 4081 | 4087 | 4093 | 4111 | 4150 |
| | | | | | | 4180 | 4252 | 4261 | 4279 | 4300 | 4354 |
| | | | | | | 4393 | 4468 | 4498 | 4531 | 4549 | 4636 |
| | | | | | | 4642 | 4720 | 4765 | 4828 | 4882 | 4888 |
| | | | | | | 4897 | 4945 | 4957 | 4972 | 4981 | 4987 |

T O T A L :  39          T O T A L :  84

| | | | | | | \*\*\*\*\* FRAME 2 \*\*\*\*\* | | | | | |
|---|---|---|---|---|---|---|---|---|---|---|---|
| 131 | 539 | 593 | 815 | 1052 | 1202 | 158 | 266 | 440 | 443 | 1058 | 1259 |
| 1205 | 3539 | 3833 | 3875 | 3986 | 4040 | 1301 | 1310 | 1433 | 1529 | 1574 | 1613 |
| 4097 | 4385 | | | | | 1616 | 1631 | 1634 | 1649 | 1670 | 1709 |
| | | | | | | 1727 | 1730 | 1751 | 1757 | 1763 | 1778 |
| | | | | | | 1853 | 1856 | 1868 | 1877 | 1982 | 2009 |
| | | | | | | 2060 | 2258 | 2372 | 2384 | 2417 | 2423 |
| | | | | | | 2444 | 2495 | 2519 | 2528 | 2567 | 2573 |
| | | | | | | 2627 | 2705 | 2717 | 2747 | 2783 | 2792 |
| | | | | | | 2810 | 2843 | 2903 | 2906 | 2924 | 2951 |
| | | | | | | 2969 | 2972 | 2993 | 3047 | 3086 | 3089 |
| | | | | | | 3110 | 3134 | 3149 | 3179 | 3209 | 3230 |
| | | | | | | 3233 | 3236 | 3272 | 3299 | 3323 | 3344 |
| | | | | | | 3386 | 3389 | 3443 | 3464 | 3476 | 3485 |
| | | | | | | 3488 | 3503 | 3506 | 3518 | 3521 | 3578 |
| | | | | | | 3602 | 3620 | 3662 | 3665 | 3671 | 3686 |
| | | | | | | 3689 | 3701 | 3749 | 3755 | 3761 | 3767 |
| | | | | | | 3788 | 3818 | 3944 | | | |

T O T A L :  14          T O T A L :  99

| | | | | | | \*\*\*\*\* FRAME 3 \*\*\*\*\* | | | | | |
|---|---|---|---|---|---|---|---|---|---|---|---|
| 27 | 282 | 483 | 522 | 555 | 693 | 75 | 168 | 189 | 258 | 447 | 540 |
| 741 | 744 | 774 | 930 | 1194 | 1275 | 732 | 1053 | 1203 | 1206 | 1401 | 1458 |
| 1287 | 1476 | 1599 | 1656 | 1770 | 1800 | 2097 | 2133 | 2193 | 2313 | 2331 | 2343 |
| 1845 | 1947 | 2292 | 2403 | 2667 | 2727 | 3537 | 3540 | 3798 | 3894 | 3900 | 3984 |
| 2787 | 2826 | 2997 | 3165 | 3285 | 3636 | 3987 | 4038 | 4275 | 4386 | 4455 | 4539 |
| 3651 | 3711 | 3714 | 3960 | 4110 | 4128 | 4686 | 4992 | | | | |
| 4179 | 4254 | 4263 | 4299 | 4305 | 4353 | | | | | | |
| 4446 | 4620 | 4722 | 4932 | 4944 | | | | | | | |

T O T A L :  47          T O T A L :  32

*** MOLECULAR WEIGHT ***                     Fig. 5 (S. 2)

##### ***** FRAME 1 *****

| START | END | MOLECULAR WEIGHT | NO. | START | END | MOLECULAR WEIGHT |
|---|---|---|---|---|---|---|
| 157 | 286 | 4842.59 | 1 | 3046 | 3418 | 13607.00 |
| 331 | 481 | 5857.77 | 2 | 3133 | 3418 | 10646.71 |
| 433 | 481 | 1911.04 | 3 | 2428 | 2665 | 9020.45 |
| 1060 | 1171 | 4219.95 | 4 | 1435 | 1675 | 8907.70 |
| 1309 | 1339 | 1055.14 | 5 | 2479 | 2665 | 7032.09 |
| 1435 | 1675 | 8907.70 | 6 | 1483 | 1675 | 6959.42 |
| 1483 | 1675 | 6959.42 | 7 | 3235 | 3418 | 6919.29 |
| 1669 | 1675 | 262.36 | 8 | 2839 | 3004 | 6213.38 |
| 1729 | 1735 | 305.39 | 9 | 331 | 481 | 5857.77 |
| 2059 | 2125 | 2514.97 | 10 | 3721 | 3868 | 5519.15 |
| 2377 | 2404 | 1011.23 | 11 | 157 | 286 | 4842.59 |
| 2383 | 2404 | 750.93 | 12 | 3883 | 4009 | 4697.94 |
| 2428 | 2665 | 9020.45 | 13 | 3292 | 3418 | 4696.55 |
| 2479 | 2665 | 7032.09 | 14 | 3748 | 3868 | 4490.94 |
| 2620 | 2665 | 1563.81 | 15 | 1060 | 1171 | 4219.95 |
| 2719 | 2728 | 375.51 | 16 | 3619 | 3712 | 3648.43 |
| 2782 | 2788 | 305.39 | 17 | 3487 | 3559 | 2767.29 |
| 2839 | 3004 | 6213.38 | 18 | 2059 | 2125 | 2514.97 |
| 2971 | 3004 | 1408.75 | 19 | 3661 | 3712 | 1946.41 |
| 2992 | 3004 | 578.66 | 20 | 433 | 481 | 1911.04 |
| 3046 | 3418 | 13607.00 | 21 | 3511 | 3559 | 1887.18 |
| 3133 | 3418 | 10646.71 | 22 | 3664 | 3712 | 1815.22 |
| 3235 | 3418 | 6919.29 | 23 | 3520 | 3559 | 1585.79 |
| 3292 | 3418 | 4696.55 | 24 | 3670 | 3712 | 1582.93 |
| 3388 | 3418 | 1294.51 | 25 | 2620 | 2665 | 1563.81 |
| 3487 | 3559 | 2767.29 | 26 | 3673 | 3712 | 1451.74 |
| 3511 | 3559 | 1887.18 | 27 | 2971 | 3004 | 1408.75 |
| 3520 | 3559 | 1585.79 | 28 | 3388 | 3418 | 1294.51 |
| 3619 | 3712 | 3648.43 | 29 | 1309 | 1339 | 1055.14 |
| 3661 | 3712 | 1946.41 | 30 | 2377 | 2404 | 1011.23 |
| 3664 | 3712 | 1815.22 | 31 | 2383 | 2404 | 750.93 |
| 3670 | 3712 | 1582.93 | 32 | 2992 | 3004 | 578.66 |
| 3673 | 3712 | 1451.74 | 33 | 3697 | 3712 | 547.68 |
| 3697 | 3712 | 547.68 | 34 | 4135 | 4150 | 535.70 |
| 3721 | 3868 | 5519.15 | 35 | 2719 | 2728 | 375.51 |
| 3748 | 3868 | 4490.94 | 36 | 1729 | 1735 | 305.39 |
| 3859 | 3868 | 277.33 | 37 | 2782 | 2788 | 305.39 |
| 3883 | 4009 | 4697.94 | 38 | 3859 | 3868 | 277.33 |
| 4135 | 4150 | 535.70 | 39 | 1669 | 1675 | 262.36 |

##### ***** FRAME 2 *****

| START | END | MOLECULAR WEIGHT | NO. | START | END | MOLECULAR WEIGHT |
|---|---|---|---|---|---|---|
| 131 | 158 | 1002.19 | 1 | 539 | 1058 | 19095.03 |
| 539 | 1058 | 19095.03 | 2 | 593 | 1058 | 17110.92 |
| 593 | 1058 | 17110.92 | 3 | 815 | 1058 | 9231.94 |
| 815 | 1058 | 9231.94 | 4 | 3833 | 3944 | 4193.88 |
| 1052 | 1058 | 236.28 | 5 | 3875 | 3944 | 2659.22 |
| 1202 | 1259 | 2324.59 | 6 | 1202 | 1259 | 2324.59 |
| 1205 | 1259 | 2193.40 | 7 | 1205 | 1259 | 2193.40 |
| 3539 | 3578 | 1532.78 | 8 | 3539 | 3578 | 1532.78 |
| 3833 | 3944 | 4193.88 | 9 | 131 | 158 | 1002.19 |
| 3875 | 3944 | 2659.22 | 10 | 1052 | 1058 | 236.28 |

Fig. 5 (S. 3)

| | | | ***** FRAME 3 ***** | | | |
|---|---|---|---|---|---|---|
| 27 | 75 | 1815.23 | 1 | 2403 | 3537 | 43550.02 |
| 282 | 447 | 5855.63 | 2 | 2667 | 3537 | 34028.78 |
| 483 | 540 | 2003.43 | 3 | 2727 | 3537 | 31646.22 |
| 522 | 540 | 616.78 | 4 | 2787 | 3537 | 29263.59 |
| 555 | 732 | 7063.49 | 5 | 2826 | 3537 | 27663.74 |
| 693 | 732 | 1636.98 | 6 | 1476 | 2097 | 24121.86 |
| 741 | 1053 | 11799.97 | 7 | 2997 | 3537 | 21162.74 |
| 744 | 1053 | 11668.78 | 8 | 1599 | 2097 | 19440.48 |
| 774 | 1053 | 10510.39 | 9 | 1656 | 2097 | 17213.04 |
| 930 | 1053 | 4670.61 | 10 | 3165 | 3537 | 14339.49 |
| 1194 | 1203 | 334.43 | 11 | 1770 | 2097 | 12848.35 |
| 1275 | 1401 | 4929.75 | 12 | 741 | 1053 | 11799.97 |
| 1287 | 1401 | 4398.11 | 13 | 744 | 1053 | 11668.78 |
| 1476 | 2097 | 24121.86 | 14 | 1800 | 2097 | 11612.94 |
| 1599 | 2097 | 19440.48 | 15 | 774 | 1053 | 10510.39 |
| 1656 | 2097 | 17213.04 | 16 | 4722 | 4992 | 10224.42 |
| 1770 | 2097 | 12848.35 | 17 | 1845 | 2097 | 9839.93 |
| 1800 | 2097 | 11612.94 | 18 | 3285 | 3537 | 9689.43 |
| 1845 | 2097 | 9839.93 | 19 | 555 | 732 | 7063.49 |
| 1947 | 2097 | 5672.19 | 20 | 4110 | 4275 | 6361.00 |
| 2292 | 2313 | 766.90 | 21 | 3636 | 3798 | 6115.51 |
| 2403 | 3537 | 43550.02 | 22 | 282 | 447 | 5855.63 |
| 2667 | 3537 | 34028.78 | 23 | 4128 | 4275 | 5690.14 |
| 2727 | 3537 | 31646.22 | 24 | 1947 | 2097 | 5672.19 |
| 2787 | 3537 | 29263.59 | 25 | 3651 | 3798 | 5533.90 |
| 2826 | 3537 | 27663.74 | 26 | 1275 | 1401 | 4929.75 |
| 2997 | 3537 | 21162.74 | 27 | 930 | 1053 | 4670.61 |
| 3165 | 3537 | 14339.49 | 28 | 1287 | 1401 | 4398.11 |
| 3285 | 3537 | 9689.43 | 29 | 4179 | 4275 | 3784.82 |
| 3636 | 3798 | 6115.51 | 30 | 4299 | 4386 | 3385.17 |
| 3651 | 3798 | 5533.90 | 31 | 3711 | 3798 | 3278.61 |
| 3711 | 3798 | 3278.61 | 32 | 3714 | 3798 | 3147.42 |
| 3714 | 3798 | 3147.42 | 33 | 4305 | 4386 | 3140.83 |
| 3960 | 3984 | 918.13 | 34 | 4620 | 4686 | 2646.26 |
| 4110 | 4275 | 6361.00 | 35 | 4932 | 4992 | 2321.86 |
| 4128 | 4275 | 5690.14 | 36 | 483 | 540 | 2003.43 |
| 4179 | 4275 | 3784.82 | 37 | 4944 | 4992 | 1867.26 |
| 4254 | 4275 | 923.20 | 38 | 27 | 75 | 1815.23 |
| 4263 | 4275 | 522.68 | 39 | 693 | 732 | 1636.98 |
| 4299 | 4386 | 3385.17 | 40 | 4353 | 4386 | 1350.57 |
| 4305 | 4386 | 3140.83 | 41 | 4254 | 4275 | 923.20 |
| 4353 | 4386 | 1350.57 | 42 | 3960 | 3984 | 918.13 |
| 4446 | 4455 | 395.51 | 43 | 2292 | 2313 | 766.90 |
| 4620 | 4686 | 2646.26 | 44 | 522 | 540 | 616.78 |
| 4722 | 4992 | 10224.42 | 45 | 4263 | 4275 | 522.68 |
| 4932 | 4992 | 2321.86 | 46 | 4446 | 4455 | 395.51 |
| 4944 | 4992 | 1867.26 | 47 | 1194 | 1203 | 334.43 |

Fig. 6 (S. 1)

***     INITIATION/TERMINATION REFERENCE     ***

| INITIATION POSITION | | | | | | TERMINATION POSITION | | | | | |
|---|---|---|---|---|---|---|---|---|---|---|---|
| ***** | FRAME 1 | ***** | | | | | | | | | |
| 514 | 1399 | 3946 | | | | 187 | 211 | 262 | 292 | 331 | 379 |
| | | | | | | 430 | 553 | 568 | 583 | 652 | 694 |
| | | | | | | 700 | 727 | 754 | 766 | 778 | 820 |
| | | | | | | 871 | 874 | 880 | 991 | 1111 | 1180 |
| | | | | | | 1186 | 1255 | 1270 | 1303 | 1363 | 1369 |
| | | | | | | 1396 | 1432 | 1495 | 1678 | 1699 | 1735 |
| | | | | | | 1768 | 1789 | 1792 | 1822 | 1987 | 2014 |
| | | | | | | 2146 | 2173 | 2233 | 2272 | 2356 | 2425 |
| | | | | | | 2431 | 2479 | 2536 | 2566 | 2581 | 2626 |
| | | | | | | 2647 | 2812 | 2920 | 2923 | 2977 | 2989 |
| | | | | | | 3016 | 3052 | 3076 | 3202 | 3229 | 3235 |
| | | | | | | 3247 | 3274 | 3280 | 3355 | 3367 | 3424 |
| | | | | | | 3505 | 3523 | 3544 | 3565 | 3649 | 3652 |
| | | | | | | 3700 | 3718 | 3730 | 3760 | 3769 | 3778 |
| | | | | | | 3805 | 3808 | 3922 | 3928 | 3940 | 4042 |
| | | | | | | 4069 | 4078 | 4087 | 4102 | 4123 | 4258 |
| | | | | | | 4306 | 4438 | 4495 | 4621 | 4657 | 4675 |
| | | | | | | 4684 | 4816 | | | | |
| T O T A L :   3 | | | | | | T O T A L :   104 | | | | | |
| ***** | FRAME 2 | ***** | | | | | | | | | |
| 203 | 863 | 896 | 1115 | 1139 | 1172 | 17 | 26 | 59 | 62 | 110 | 164 |
| 1685 | 1730 | 2378 | 2495 | 2621 | 2642 | 245 | 278 | 299 | 347 | 350 | 404 |
| 3569 | 3875 | 4427 | 4778 | | | 449 | 470 | 530 | 578 | 626 | 719 |
| | | | | | | 746 | 749 | 782 | 797 | 800 | 839 |
| | | | | | | 842 | 1142 | 1166 | 1466 | 1781 | 1994 |
| | | | | | | 2261 | 2393 | 2498 | 2543 | 2837 | 2945 |
| | | | | | | 3323 | 3530 | 3881 | 3968 | 3977 | 3983 |
| | | | | | | 3986 | 4034 | 4049 | 4052 | 4082 | 4157 |
| | | | | | | 4184 | 4187 | 4190 | 4193 | 4202 | 4211 |
| | | | | | | 4220 | 4334 | 4397 | 4433 | 4502 | 4529 |
| | | | | | | 4532 | 4535 | 4538 | 4742 | 4859 | 4862 |
| T O T A L :   16 | | | | | | T O T A L :   66 | | | | | |
| ***** | FRAME 3 | ***** | | | | | | | | | |
| 870 | 873 | 1002 | 1269 | 1287 | 1362 | 372 | 459 | 522 | 543 | 639 | 723 |
| 1542 | 1677 | 1800 | 2172 | 2646 | 3114 | 864 | 1044 | 1173 | 1200 | 1380 | 1563 |
| 3153 | 3639 | 3675 | 3699 | 3816 | 4716 | 1575 | 1614 | 1662 | 1686 | 1707 | 1752 |
| | | | | | | 1797 | 1872 | 1896 | 1923 | 1953 | 2007 |
| | | | | | | 2019 | 2097 | 2139 | 2160 | 2166 | 2244 |
| | | | | | | 2307 | 2319 | 2379 | 2457 | 2508 | 2571 |
| | | | | | | 2643 | 2667 | 2865 | 3069 | 3111 | 3159 |
| | | | | | | 3183 | 3330 | 3351 | 3450 | 3465 | 3477 |
| | | | | | | 3516 | 3540 | 3597 | 3681 | 3690 | 3705 |
| | | | | | | 3786 | 3876 | 4266 | 4284 | 4779 | 4809 |
| | | | | | | 4821 | 4923 | | | | |
| T O T A L :   18 | | | | | | T O T A L :   62 | | | | | |

*** MOLECULAR WEIGHT ***         Fig. 6 (S. 2)

| START | END | MOLECULAR WEIGHT | NO. | START | END | MOLECULAR WEIGHT |
|---|---|---|---|---|---|---|
| ***** FRAME 1 ***** | | | | | | |
| 514 | 553 | 1629.87 | 1 | 3946 | 4042 | 3550.03 |
| 1399 | 1432 | 1344.47 | 2 | 514 | 553 | 1629.87 |
| 3946 | 4042 | 3550.03 | 3 | 1399 | 1432 | 1344.47 |
| ***** FRAME 2 ***** | | | | | | |
| 203 | 245 | 1707.87 | 1 | 3569 | 3881 | 11635.17 |
| 863 | 1142 | 10853.68 | 2 | 1172 | 1466 | 11262.47 |
| 896 | 1142 | 9431.19 | 3 | 863 | 1142 | 10853.68 |
| 1115 | 1142 | 1115.33 | 4 | 896 | 1142 | 9431.19 |
| 1139 | 1142 | 149.21 | 5 | 2621 | 2837 | 7747.28 |
| 1172 | 1466 | 11262.47 | 6 | 2642 | 2837 | 6866.30 |
| 1685 | 1781 | 3768.36 | 7 | 1685 | 1781 | 3768.36 |
| 1730 | 1781 | 1988.33 | 8 | 4778 | 4859 | 3218.74 |
| 2378 | 2393 | 656.78 | 9 | 1730 | 1781 | 1988.33 |
| 2495 | 2498 | 149.21 | 10 | 203 | 245 | 1707.87 |
| 2621 | 2837 | 7747.28 | 11 | 1115 | 1142 | 1115.33 |
| 2642 | 2837 | 6866.30 | 12 | 2378 | 2393 | 656.78 |
| 3569 | 3881 | 11635.17 | 13 | 3875 | 3881 | 277.38 |
| 3875 | 3881 | 277.38 | 14 | 4427 | 4433 | 246.32 |
| 4427 | 4433 | 246.32 | 15 | 1139 | 1142 | 149.21 |
| 4778 | 4859 | 3218.74 | 16 | 2495 | 2498 | 149.21 |
| ***** FRAME 3 ***** | | | | | | |
| 870 | 1044 | 6984.76 | 1 | 870 | 1044 | 6984.76 |
| 873 | 1044 | 6853.57 | 2 | 873 | 1044 | 6853.57 |
| 1002 | 1044 | 1764.94 | 3 | 1269 | 1380 | 4059.50 |
| 1269 | 1380 | 4059.50 | 4 | 1287 | 1380 | 3436.80 |
| 1287 | 1380 | 3436.80 | 5 | 2172 | 2244 | 2682.96 |
| 1362 | 1380 | 748.87 | 6 | 4716 | 4779 | 2652.92 |
| 1542 | 1563 | 879.86 | 7 | 1800 | 1872 | 2593.86 |
| 1677 | 1686 | 418.54 | 8 | 3816 | 3876 | 2182.40 |
| 1800 | 1872 | 2593.86 | 9 | 3114 | 3159 | 1826.12 |
| 2172 | 2244 | 2682.96 | 10 | 1002 | 1044 | 1764.94 |
| 2646 | 2667 | 838.92 | 11 | 3639 | 3681 | 1629.97 |
| 3114 | 3159 | 1826.12 | 12 | 1542 | 1563 | 879.86 |
| 3153 | 3159 | 248.34 | 13 | 2646 | 2667 | 838.92 |
| 3639 | 3681 | 1629.97 | 14 | 1362 | 1380 | 748.87 |
| 3675 | 3681 | 206.26 | 15 | 1677 | 1686 | 418.54 |
| 3699 | 3705 | 236.28 | 16 | 3153 | 3159 | 248.34 |
| 3816 | 3876 | 2182.40 | 17 | 3699 | 3705 | 236.28 |
| 4716 | 4779 | 2652.92 | 18 | 3675 | 3681 | 206.26 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | WO-A-84 001 107 (THE GENERAL HOSPITAL CORP.) <br> * Insgesamt * <br> --- | 1-10 | C 12 N  15/00 <br> A 61 K  39/29 <br> C 12 Q  1/68 <br> G 01 N  33/576 |
| X | EP-A-0 124 896  (SEELIG) <br> * Beispiel 7 * <br> --- | 7 | |
| A | EP-A-0 066 296  (EISAI CO., LTD) <br> --- | | |
| A | WO-A-82 003 330 (TREPO) <br> --- | | |
| A | RECHERCHE, Band 14, Nr. 145, Juni 1983, Seiten 854-865, Paris, FR; A. ZOTOV: "Les hépatites" <br> --- | | |
| P,A | EP-A-0 242 300  (INSTITUT PASTEUR) <br> ----- | | |

RECHERCHIERTE SACHGEBIETE (Int. Cl.4)

C 12 N
A 61 K

**Der vorliegende Recherchenbericht wurde für alle Patentansprüche erstellt**

| Recherchenort | Abschlußdatum der Recherche | Prüfer |
|---|---|---|
| DEN HAAG | 29-04-1988 | SKELLY J.M. |